(19) Europäisches Patentamt / European Patent Office / Office européen des brevets

(11) EP 3 399 027 A1

(12) EUROPEAN PATENT APPLICATION

(43) Date of publication:
07.11.2018 Bulletin 2018/45

(51) Int Cl.:
C12N 5/078 (2010.01)        C12N 5/0787 (2010.01)
C12N 5/0786 (2010.01)        A61K 35/15 (2015.01)

(21) Application number: 17169454.0

(22) Date of filing: 04.05.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Medizinische Hochschule Hannover
30625 Hannover (DE)

(72) Inventors:
• LACHMANN, Nico
  30171 Hannover (DE)
• ACKERMANN, Mania
  30169 Hannover (DE)
• ZWEIGERDT, Robert
  30559 Hannover (DE)
• KEMPF, Henning
  30173 Hannover (DE)
• MORITZ, Thomas
  53177 Bonn (DE)

(74) Representative: Moré, Solveig Helga et al
Kroher Strobel
Rechts- und Patentanwälte PartmbB
Bavariaring 20
80336 München (DE)

(54) STEM-CELL DERIVED MYELOID CELLS, GENERATION AND USE THEREOF

(57) The present invention relates to stem-cell derived hematopoietic cells, in particular, myeloid cells, preferably, macrophages, their generation and use. In particular, the invention relates to a method of producing hematopoietic, preferably, myeloid cells, comprising cultivating embryoid bodies, which are, e.g., derivable from pluripotent stem cells such as induced pluripotent stem cells (iPSC), in suspension culture, to produce myeloid cell forming complexes, which are further cultivated in suspension culture to produce myeloid cells such as macrophages. This allows for a scalable and continuous production, e.g., in industry-compatible stirred tank bioreactors. Macrophages, e.g., macrophages produced with this method that have unique characteristics, can be used in pharmaceutical compositions for treatment of patients, e.g., for treatment of infection such as bacterial infection. The invention further provides application systems suitable for spraying, comprising myeloid cells such as macrophages for use in treatment of patients for treatment of infection, e.g., bacterial infection or wound healing.

Fig 2

A
hiPSC culture → d-5 → EB formation → d0 → Hematopoietic specification → d10-15 → Macrophage generation → Harvest
1  2  3  4  n ......

EP 3 399 027 A1

**Description**

The present invention relates to stem-cell derived hematopoietic cells, in particular, myeloid cells, preferably, macrophages, their generation and use. In particular, the invention relates to a method of producing hematopoietic, preferably, myeloid cells, comprising cultivating embryoid bodies, which are, e.g., derivable from pluripotent stem cells such as induced pluripotent stem cells (iPSC), in suspension culture, to produce myeloid cell forming complexes, which are further cultivated in suspension culture to produce myeloid cells such as macrophages, preferably, in a continuous manner. This allows for a scalable and continuous production, e.g., in industry-compatible stirred tank bioreactors. Macrophages, e.g., macrophages produced with this method that have unique characteristics, can be used in pharmaceutical compositions for treatment of patients, e.g., for treatment of infection such as bacterial infection. The invention further provides application systems suitable for spraying, comprising myeloid cells such as macrophages for use in treatment of patients for treatment of infection, e.g., bacterial infection or wound healing.

The increasing number of severe infections with multi-drug-resistant pathogens worldwide highlights the need for alternative treatment options. The fact that the respective pathogens are often refractory not only to standard antibiotic therapy but even resistant against drugs of last resort creates a severe clinical problem. Thus, alternative strategies targeting bacterial infections are urgently required (Freire-Moran et al., 2011; Ventola, 2015; Willyard, 2017).

One promising alternative to antibiotic therapy might be a cell based treatment approach applying stem cell-derived immune cells, in particular phagocytes as an important component of the innate immune system. As of yet, however, generating the therapeutically required amounts of phagocytes from peripheral blood or other sources is challenging. In contrast to somatic cell sources and more specified adult hematopoietic stem cells, human pluripotent stem cells (hPSCs), with their unlimited potential for proliferation and differentiation, may - in principle - enable this therapeutic scenario. In this line, hematopoietic differentiation of human PSC has been proven feasible (Ackermann et al., 2015) and has been proposed as a promising strategy for future cell-based treatment approaches. However, clinical translation of hPSC-derived hematopoiesis remains hampered by insufficient knowledge about *in vivo* functionality and the lack of therapeutically relevant quantities of effector cells.

The inventors have recently shown that the potential of human embryonic stem cells (hESCs) and induced pluripotent stem cells (hiPSCs) for unlimited proliferation and targeted differentiation is not only a theoretical feature, but can be put into practice by developing scalable and potentially GMP-compliant cell production processes in fully controlled stirred tank bioreactors (Kempf et al., 2015; Kropp et al., 2016a; Zweigerdt et al., 2011). While feasibility of the approach was demonstrated for hPSC expansion (Kropp et al., 2016b; Olmer et al., 2012) and cardiomyogenic differentiation in stirred suspension (Kempf et al., 2016; Kempf et al., 2015; Kempf et al., 2014), the successful use of bioreactor systems for generating hematopoietic cells from multipotent or pluripotent stem cell sources is yet elusive. Similar observations have been made for the *in vivo* applicability of PSC-derived hematopoietic cells. Although the generation of mature immune cells including phagocytes from human PSC has been proven (Buchrieser et al., 2017; Lachmann et al., 2015; van Wilgenburg et al., 2013; Zhang et al., 2015), data on their actual *in vivo* functionality remains as of yet scarce.

In light of the state of the art, the inventors addressed the problem of providing a method of producing hematopoietic, in particular, myeloid cells in large quantities, preferably, allowing for continuous production. This problem is solved by the present invention, in particular, by the subject matter of the claims.

The present invention thus provides a method of producing hematopoietic, in particular, myeloid cells, comprising steps of

   a) cultivating embryoid bodies in suspension culture in the presence of IL-3 and, optionally, at least one additional cytokine, for a sufficient period of time to produce myeloid cell forming complexes;

   b) cultivating the myeloid cell forming complexes in the presence of IL-3 and, optionally, at least one additional cytokine, in suspension culture for a sufficient period of time to produce myeloid cells; and

   c) isolating the myeloid cells.

Said method allows for continuous production, and, preferably, it is used for continuous production of myeloid cells.

Embryoid bodies (EBs) are three-dimensional aggregates derived from pluripotent stem cells (PSC), which are typically rounded and comprise cell types of all three germ layers. PSC are undifferentiated cells that can differentiate into specialized cells, in particular, they are capable of differentiation into cells of all three germ layers under suitable conditions. Pluripotent cell types from which embryoid bodies may be derived include embryonic stem cells (ESCs) derived from the blastocyst stage of embryos, e.g., from mouse (mESC), primate, and human (hESC) sources.

Additionally, EBs can be formed from PSC derived through alternative techniques, including somatic cell nuclear transfer or the reprogramming of somatic cells to yield induced pluripotent stem cells (iPSC). Methods for generating EBs are well known in the art.

In the context of the present invention, EBs are preferably not derived from stem cells for generation of which destruction of human embryos was required. Preferably, the EBs are derived from iPSC.

Similar to stem cells differentiated in monolayer formats, stem cells within embryoid bodies undergo differentiation and cell specification along the three germ lineages - endoderm, ectoderm, and mesoderm - which comprise all somatic cell types.

In contrast to monolayer differentiation cultures, however, the spheroid structures that are formed when PSCs aggregate enable the non-adherent culture of EBs in suspension. This makes EB cultures inherently scalable, which is useful for bioprocessing approaches, whereby large yields of cells can be produced for potential clinical applications. Additionally, although EBs largely exhibit heterogeneous patterns of differentiated cell types, they are capable of responding to similar cues as those that direct embryonic development.

As stem cells may undergo apoptosis when cultured as single cells, EB formation often necessitates the use of inhibitors of the rho associated kinase (ROCK) pathway, e.g., Y-27632, HA-100, H-1152 and/or 2,4 disubstituted thiazole (Thiazovivin/Tzv), preferably, Y-27632, e.g., about 10 $\mu$M. Alternatively, to avoid dissociation into single cells, EBs can be formed from PSCs such as hiPSCs by manual separation of adherent colonies (or regions of colonies) or, preferably, by enzymatic treatment with collagenase IV, and subsequently cultured in suspension. Formation of EBs in suspension allows for the formation of large quantities of EBs, but provides little control over the size of the resulting aggregates, often leading to large, irregularly shaped EBs. Mixed (or moved, e.g., stirred or shaken) culture platforms increase the homogeneity of EB sizes when ESCs are inoculated within bulk suspensions.

Preferably, the embryoid bodies of the invention are obtained by a method comprising cultivating pluripotent stem cells such as iPSC in suspension culture for a sufficient period of time to produce embryoid bodies. For therapeutic uses, in particular, for use in humans, use of feeder cells should be avoided. This can be compensated for by adding cytokines e.g. SCF, BMP4, VEGF or small molecules including WNT pathway modulators such as CHIR99021 ((6-[[2-[[4-(2,4-Dichlorophenyl)-5-(5-methyl-1H-imidazol-2-yl)-2-pyrimidinyl] amino] ethyl]amino]-3-pyridinecarbonitrile (TOCRIS)) and BIO ((2'Z,3'E)-6-Bromoindirubin-3'-oxime) (TOCRIS)). Preferably, the culture conditions include use of ROCK inhibitor, but addition of bFGF is not required.

Suitable embryoid bodies for use in the methods of the present invention can be selected manually, but, preferably, they are selected according to their sedimentation characteristics. For example, EBs which sediment (without centrifugation) within 10 min, preferably, within 5 min, may be used (WO2010/025776A1).

The pluripotent stem cells from which the EB are derived, and to which, consequently the produced myeloid cells belong, may be from any species, e.g., mouse, rat, rabbit, guinea pig, cat, horse, dog, cow, camel, pig, sheep, goat, monkey or primate, e.g., human. Human cells are preferred. In case the cells are intended for treatment of a human, human myeloid cells such as macrophages are preferably produced. In the method of the invention, preferably, cytokines from the relevant species are used. These may be recombinant cytokines, optionally, comprising mutations, which do not diminish their functionality in the differentiation processes involved.

The suspension culture of the invention prevents adhesion to surfaces, in particular to surfaces of containers. The suspension culture also does not allow adhesion to carriers. Preferably, the suspension culture is moved to prevent adherence or sedimentation of cells, e.g., shaken, rotated or stirred. The culture may take place in suspension plates, e.g., on an orbital shaker, or in roller bottles coated to prevent adhesion.

In a preferred embodiment, the suspension culture is carried out in a bioreactor allowing suspension culture such as an Erlenmeyer flask, spinner flask, stirred tank bioreactor, wave bioreactor and rotating wall bioreactor, preferentially in a stirred tank bioreactor, most preferably in an instrumented stirred tank bioreactor, i.e., a bioreactor equipped with technologies to monitor and control process parameters such as pH, pO2, temperature and stirring speed. Such systems are scalable, i.e., the volume of the culture can be changed without significantly changing culture conditions. The DASbox Mini bioreactor system, Eppendorf may be used, e.g., as described below.

For compatibility with industrial processes, relative linear upscaling is probably the most important criteria. Preferred systems may already be utilized in the industry for other purposes, and may fulfil industry standards, e.g. they may be equipped with standard ports that allow incorporation of standard probes for process monitoring and control, and established sterilization and equipment validation procedures. Bioreactors may be GMP-compatible.

Bioreactors used in the invention, e.g., stirred tank bioreactors, may be equipped with ports enabling continuous sampling, monitoring and harvest of cells, in particular monocytes and macrophages. They may also be equipped with suitable cell retention systems allowing perfusion-based cell feeding and online monitoring of culture medium components such as metabolites e.g. glucose, lactate and ammonium. The bioreactor may be a single-use (disposable) or reusable bioreactor, utilising glass, plastic or stainless steel vessels.

Preferably, the cultivation in a stirred tank bioreactor is carried out at about 37°C, with headspace gassing at about 3L/h with about 21% $O_2$, about 5% $CO_2$, and stirring at about 50 revolutions per minute using a multiple blade (e.g., 4-12 blade)-pitched impeller. In the context of the invention, "about" means +/- 10%, preferably, +/- 5% or +/- 2%.

The cultivation may be at 37°C, with headspace gassing at 3L/h with 21% $O_2$, 5% $CO_2$, and stirring at 50 revolutions per minute using an 8 blade-pitched 60°C impeller and, optionally, with real-time monitoring of dissolved oxygen, pH, temperature, and impedance-based biomass assessment.

Preferably, the method of the invention allows for continuous production of myeloid cells, which includes repeated batch methods. Continuous production *strictu sensu* is also possible, and may allow for more efficacious cell production. For example, a cell retention system enabling perfusion may be used in combination with continuous medium replacement to optimize culture conditions.

The volume of the suspension culture, e.g., in the stirred tank bioreactor, is scalable, e.g., 50 mL - 1000 L, 100 mL - 500 L, 200 mL - 100 L, 500 mL to 50 L or 1 L-20 L. Preferably, in particular for therapeutic application of the produced cells, the medium used for cultivation is a chemically defined medium compatible with application in humans, e.g., X-VIVO 15 (Lonza) or APEL (Stem Cell Technologies), preferably, X-VIVO 15.

Myeloid cell forming complexes (MCFC) are known in the art (Lachmann et al., 2015). They contain CD34+ clonogenic progenitor cells. They are capable of continuously producing myeloid cells. In the presence of IL-3, myeloid cells are delivered into the culture medium. Of note, the method of the present invention does not require any purification of MCFC, it just requires that MCFC are formed in step a) and that they produce myeloid cells in step b). Step a) and b) (and, optionally, even c)) can overlap, i.e., it is possible that further EB differentiate into MCFC while other MCFC already produce myeloid cells (which may already be isolated).

Generation of MCFC from EBs typically takes about 4-8 days. Generation of first myeloid cells from MCFC starts after formation of the MCFC, and continuous generation is observed thereafter. Typically, harvest is started about 3-16 days after generation of MCFC. A suitable time for production of MCFC from EB and production of myeloid cells together (i.e., step a) and step b) together) is at least about 7 days, e.g., 7-20 days. The inventors could show that, from day 7-14 onwards, at least weekly harvest of macrophages from a stirred bioreactor system is possible, showing an increasing yield over time. In a 250mL bioreactor (120mL culture volume), stable production of about $2-3x10^7$ macrophages/week is possible as early as week three, which can be maintained over time for at least 5 weeks. Of course, cultivation times and conditions may be varied depending on the phenotype of the desired cells. For example, harvesting continuously or in in small intervals may lead to production of less mature cells. The harvested cells can than, optionally, as discussed later below, be subject to further maturation.

Culture in the presence of a cytokine such as IL-3 does not necessitate continuous presence of said cytokine. It is for example possible to culture the cells in the absence of IL-3 for time periods, but differentiation and, in particular, production of myeloid cells by MCFC requires presence of IL-3. Suitable amounts or IL-3 are, e.g., 10-100 ng/mL, preferably, 20-30 ng/mL (most preferably, about 25 ng/ml) IL-3.

For formation of MCFC, differentiation medium I may be used, e.g., a basic medium, preferably, a chemically defined medium such as X-VIVO 15 (Lonza) or APEL (Stem Cell Technologies), both suitable, e.g., for human cells, X-VIVO 15 is preferred further comprising suitable amounts, e.g., 10-100 ng/mL, preferably, 20-30 ng/mL (most preferably, about 25 ng/ml) IL-3 (obtainable, e.g., from PeproTech) and, optionally, at least one other cytokine, e.g., 40-60 ng/ml (preferably, about 50 ng/ml) M-CSF (obtainable, e.g., from PeproTech), if macrophages are to be produced. The medium may optionally comprise suitable amounts of antibiotics, e.g., 1% penicillin-streptomycin.

If the produced cells are not for use in humans, other basic media may be used, e.g., for mouse cells, RPMI1640 supplemented with 10% FCS, 2mM L-glutamine, optionally, 1% penicillin-streptomycin and at least one cytokine.

For production of myeloid cells having a mature phenotype, additional cytokines may be added at least in step b), optionally, also in step a) of the method of the invention.

In a preferred method of the invention, the additional cytokine is M-CSF (in a suitable concentration, e.g., 40-60 ng/mL, preferably, about 50 ng/mL M-CSF), and the produced myeloid cells are macrophages.

Alternatively, the additional cytokine is G-CSF (in a suitable concentration, e.g., 40-60 ng/mL, preferably, about 50 ng/mL G-CSF) and the produced myeloid cells are granulocytic precursor, which can be further differentiated towards granulocytes upon culture in G-CSF only (in a suitable concentration, e.g., 50-200 ng/mL, preferably, about 100 ng/mL G-CSF).

Alternatively, the additional cytokine is GM-CSF (in a suitable concentration, e.g., 40-60 ng/mL, preferably, about 50 ng/mL GM-CSF) and the produced myeloid cells are macrophages and granulocytic precursor, which can be further differentiated towards granulocytes upon culture in GM-CSF only (in a suitable concentration, e.g., 50-200 ng/mL, preferably, about 100 ng/mL GM-CSF).

If the additional cytokines are SCF and EPO (in suitable concentrations, e.g., 80-120 ng/mL, preferably, about 100 ng/mL SCF and 2-4 Units (U), preferably, about 3 U EPO), the produced myeloid cells comprise erythroid cells, which can be further differentiated upon culture in SCF and EPO only (in a suitable concentration, e.g., 50-200 ng/mL SCF, preferably, about 100 ng/mL SCF, and 2-4 Units (U) EPO, preferably, about 3 U EPO).

Other lineage-instructive cytokines may be applied to further broaden the spectrum of generated cell types towards dendritic cells or thrombocytes (Ackermann et al., 2015; Choi et al., 2009; Lachmann et al., 2015; Ma et al., 2008).

Alternatively, the additional cytokines are IL-4 and GM-CSF (in a suitable concentration, e.g., 40-60 ng/mL, preferably, about 50 ng/mL GM-CSF and IL-4) and the produced myeloid cells comprise dendritic cells, which can be further differentiated upon culture in GM-CSF/IL4 only (in a suitable concentration, e.g., 50-200 ng/mL, preferably, about 100 ng/mL GM-CSF/IL4).

Optionally, the additional cytokines are SCF and TPO (in suitable concentrations, e.g., 80-120 ng/mL, preferably, about 100 ng/mL SCF and 2-4U, preferably, about 3 U TPO) and the produced myeloid cells comprise megakaryocytes and/or thrombocytes, which can be further differentiated upon culture in SCF/TPO only (in a suitable concentration, e.g., 50-200 ng/mL SCF, preferably, about 100 ng/mL SCF, and 2-4 Units (U) TPO, preferably, about 3 U TPO). Since IL-3 might exert a negative effect on the maturation of megakaryocytes, it might be reduced or omitted after step a).

In one embodiment of the method of the invention, no cytokine additional to IL-3 is added in either step a) or step b), and the produced myeloid cells are immature myeloid cells capable of further differentiation. Optionally, in said case, said method further comprises

i) cultivating said immature cells in the presence of M-CSF (in a suitable concentration, e.g., 40-100 ng/mL, preferably, about 50 ng/mL M-CSF) until macrophages are obtained; or

ii) cultivating said immature cells in the presence of G-CSF (in a suitable concentration, e.g., 40-120 ng/mL, preferably, about 100 ng/mL G-CSF) until granulocytes are obtained;

iii) cultivating said immature cells in the presence of GM-CSF (in a suitable concentration, e.g., 40-120 ng/mL, preferably, about 100 ng/mL GM-CSF) until granulocytes and macrophages are obtained;

iv) cultivating said immature cells in the presence of SCF and EPO (in a suitable concentration, e.g., 50-200 ng/mL SCF, preferably, about 100 ng/mL SCF, and 2-4 Units (U) EPO, preferably, about 3 U EPO) until erythroid cells are obtained;

v) cultivating said immature cells in the presence of SCF and TPO (in a suitable concentration, e.g., 50-200 ng/mL SCF, preferably, about 100 ng/mL SCF, and 2-4 Units (U) TPO, preferably, about 3 U TPO) until megakaryocytes and/or thrombocytes are obtained; or

vi) GM-CSF and IL-4 (in suitable concentrations, e.g., 40-120 ng/mL, preferably, about 100 ng/mL GM-CSF and 40-120 ng/mL IL-4, preferably, about 100 ng/mL IL-4), until dendritic cells are obtained.

In the method of the invention, isolating the produced myeloid cells comprises purifying the produced myeloid cells, preferably, the macrophages, to a purity of at least 70%, preferably, at least 80%, at least 90% or at least 95%. If the method of the invention is carried out to produce macrophages, in particular, with culture in the presence of IL-3 and M-CSF, the purity of myeloid precursor cells characterized by an expression profile of CD45$^+$CD11b$^+$CD34$^-$TRA-1-60$^-$ may be, preferably, at least 80%, at least 90% or at least 95%. At least 50%, preferably, at least 60%, or 70-90% of the produced cells are CD45$^+$CD11b$^+$CD34$^-$TRA-1-60$^-$CD14$^+$/CD163$^+$ macrophages. In one embodiment, at least 95% of the produced cells are CD45$^+$CD11b$^+$CD34$^-$TRA-1-60$^-$ and 70-90% of the produced cells are CD45$^+$CD11b$^+$CD34$^-$TRA-1-60$^-$CD14$^+$/CD163$^+$. Most of the other cells are myeloid precursors of macrophages. If the isolation is continuous or the harvests are performed in shorter intervals, the percentage of precursors may be higher.

In the method of the invention, isolating the produced myeloid cells (e.g., macrophages) may comprise, e.g., in a batch-continuous method,

ci) allowing for sedimentation of majority of the MCFC in the suspension culture, e.g., by sedimentation for about 5-12 minutes, preferably, 10 minutes (e.g., by temporarily stopping movement (e.g., stirring) of the suspension culture,

cii) removing the supernatant,

ciii) filtering the removed supernatant through a filter with a mesh of about 70-100 μm,

civ) optionally, returning the retentate (which mostly comprises MCFC) to the suspension culture, and adding new culture medium to the sedimented cells of the suspension culture,

cv) wherein the produced myeloid cells are comprised in the filtrate. Optionally, the produced myeloid cells are isolated from the filtrate by sedimentation or centrifugation. They may then be formulated for administration to a patient, e.g., be taken up in a pharmaceutically acceptable carrier, e.g., a buffer such as PBS. Alternatively, they may be further cultivated, e.g., in suspension culture, e.g. in the presence of an activating and/or maturating agent selected form the group comprising a cytokine or active agent, e.g., IL-3, M-CSF, GM-CSF, TNF-alpha, IFN-gamma or LPS, and be formulated for administration later.

Alternatively, for isolation, the medium from the suspension culture may be removed through a filter having a mesh of about 70-100 $\mu$m, wherein the produced macrophages can be isolated, e.g., by centrifugation, from the filtrate, and, optionally, formulated for administration with or without further maturation. This isolation procedure may be performed from time to time, allowing for a batch continuous production of produced cells, or continuously.

Further alternative methods for isolation of the produced myeloid cells comprise differential centrifugation or gradient centrifugation, e.g., with a sucrose gradient.

The invention thus provides a suspension culture comprising myeloid cell forming complexes which continuously produce hematopoietic cells, and, optionally, myeloid cells. Said suspension culture is, e.g., obtainable from the method of the invention above, wherein isolation of the produced myeloid cells is possible, but not required. Said suspension culture preferably is contained in a stirred tank bioreactor, e.g., as described above. In other words, the invention provides a stirred tank bioreactor comprising a suspension culture comprising myeloid cell forming complexes which continuously produce myeloid cells, and, optionally, myeloid cells. Preferably, said myeloid cells are macrophages, and the culture medium comprises IL-3 and M-CSF.

The present invention also provides a hematopoietic, preferably, a myeloid cell population obtainable by the method of the invention.

The invention e.g., provides a cell population comprising CD45$^+$CD11b$^+$CD14$^+$CD163$^+$CD34$^-$TRA1-60$^-$ macrophages, preferably, at least 50%, at least 60%, or at least 70% of said cells, which is, e.g., obtainable by the method of the invention. Analysis of expression of genes associated with pluripotency and activation of innate immune response confirmed efficient differentiation of iPSC into macrophage-like cells. Importantly, genes associated with macrophage function such as the toll-like receptors (TLR) 1 and 4, CD14, or components of the NF-$\kappa$B signaling pathway (gene ontology (GO) Activation of innate immunity: 0002218) were significantly upregulated in iPSC-MACs and PBMC-MACs versus iPSCs. Morphological analysis after adherence and functional analysis (e.g., phagocytic uptake of latex beads and bacteria) confirm that the generated cells are macrophages. The methods of the invention for the first time render it possible to produce a cell population comprising more than $1\times10^6$ macrophages, at least $2\times10^7$ macrophages, at least $5\times10^7$ macrophages, at least $1\times10^8$ macrophages, at least $1\times10^9$ macrophages or at least $1\times10^{10}$ macrophages.

The macrophages obtainable from the method of the invention typically have a significantly higher surface expression (as determined by FACS) of CD14 and C163 and a significantly lower surface expression of HLA than macrophages obtained from PBMC. The macrophages produced in the examples were shown to produce pro-inflammatory cytokines such as IL-6, IL-8, or TNF-alpha. Accordingly, they can be considered be more pro-inflammatory macrophages than anti-inflammatory macrophages. By addition of suitable cytokines e.g. IL-13, IL-10, IL-4, the phenotype can be redirected to anti-inflammatory macrophages. In addition, other substances, e.g. corticosteroide, may be used to induce an anti-inflammatory phenotype.

The inventors have found that the CD45$^+$CD11b$^+$CD14$^+$CD163$^+$CD34$^-$TRA1-60$^-$ macrophages of the invention, e.g., obtainable from the method of the invention, have a unique expression profile compared to macrophages isolated according to state of the art methods. Preferably, in the macrophages of the invention, expression of at least 10 genes selected from a group consisting of DKK1, SEPP1, PITX2, COL3A1, KRT19, A_33_P3221980, CALD1, CYR61, H19, DDIT4L, FRZB, TMEM98, NNMT, NPNT, LUM, DCN, LYVE1, MGP, IGFBP3 and NUAK1 is at least 20fold upregulated in said macrophages compared to macrophages derived from PBMC, preferably, at least 50 fold, at least 200 fold, at least 400 fold or at least 1000fold. Expression of at least 12 genes, preferably, at least 15 genes or all genes selected from a group consisting of DKK1, SEPP1, PITX2, COL3A1, KRT19, A_33_P3221980, CALD1, CYR61, H19, DDIT4L, FRZB, TMEM98, NNMT, NPNT, LUM, DCN, LYVE1, MGP, IGFBP3 and NUAK1 may be at least 100fold upregulated in said macrophages compared to macrophages derived from PBMC, preferably, at least 200 fold, at least 400 fold or at least 500 fold. Optionally, expression of CYR61, DDIT4L, KRT19, DCN, LUM, COL3A1 is at least 200 fold, at least 500 fold or at least 1000 fold upregulated in said macrophages compared to macrophages derived from PBMC.

Optionally, furthermore, in the macrophages of the invention, expression of at least 10 genes selected from a group consisting of ANPEP, CDA, CRTAM, ENST00000390237, FBP1, GBP1, GNLY, HLA_DQA1, HLA_DQA2, HLA_DQB1, HLA_DQB2, HLA_DRA, HLA_DRB1, HLA_DRB3, HLA_DRB4, HLA_DRB5, IL15, LY75, S1PR4, TNFAIP6 are at least 20fold downregulated in said macrophages compared to macrophages derived from PBMC, preferably, at least 200 fold, at least 400 fold, at least 500 fold or at least 1000 fold. Expression of at least 10, at least 12 or all genes selected from said group may be at least 100fold downregulated in said macrophages compared to macrophages derived from PBMC, preferably, at least 200 fold, at least 400 fold, at least 500 fold or at least 1000 fold.

Typical expression profiles are disclosed herein, e.g., in Table 1. It is noted that the comparison leads to particularly high differences for genes which are practically not expressed in one of the cell types compared. In that case, even very small differences in expression of the genes can lead to high differences in the comparative rate of expression. Without intending to be limited by the theory, it is believed that the shear stress in suspension culture leads to different expression of genes in the produced myeloid cells.

The invention also provides a cell population comprising at least 50%, preferably, at least 60% or at least 70% of immature CD45+CD11b+/CD14-/CD163- myeloid cells, wherein, optionally, said cell population is obtainable by the method of the invention. Said immature cells are unique due to their expression profile of CD45+CD11b+CD14-/CD163-/CD66b-/CD34$^{dim/-}$.

The invention also provides a cell population comprising at least 50%, preferably, at least 60% or at least 70% of granulocytes, wherein, optionally, said cell population is obtainable by the method of the invention. Said granulocytes are unique due to their expression profile, e.g., they express significantly less CD66b on their surface than granulocytes isolated form PBMC. Said granulocytic cells are unique due to their expression profile of CD45+CD11b+CD66b$^{dim}$, CD16$^{dim}$.

The invention also provides a cell population comprising at least 50%, preferably, at least 60% or at least 70% of erythroid cells, wherein, said cell population is obtainable by the method of the invention. Said cells appear to be erythrocyte precursors.

The invention also provides a cell population comprising at least 10%, preferably, at least 20% or at least 30% of megakaryocytes, wherein, said cell population is obtainable by the method of the invention.

The invention also provides a cell population comprising at least 10%, preferably, at least 15% or at least 20% of dendritic cells, wherein, said cell population is obtainable by the method of the invention.

Such cell population may be frozen, e.g., according to methods known in the art. They are typically thawed before use. Populations isolated, e.g., from different batches from one or more cultures at one or more time points can be combined if higher cell numbers are needed. Of particular importance could be the differentiation with only IL3 to generate a myeloid progenitor population, which can be frozen, combined with subsequent on demand differentiation towards therapeutic cell types of choice.

The present invention also provides a pharmaceutical composition comprising a cell population of the invention, preferably, a cell population comprising macrophages or immature myeloid cells of the invention, most preferably, macrophages as characterized herein, in a pharmaceutically acceptable carrier. The cell population may be obtainable from the methods of the invention as described herein.

Preferably, the cells are human cells intended for treatment of a human patient, most preferably human macrophages or immature myeloid cells of the invention, most preferably, macrophages as characterized herein.

A pharmaceutically acceptable carrier typically is a buffer, e.g., PBS (PBS/EDTA supplemented with about 20% human serum albumin, or citrate plasma, or Plasmalyte-A pH 7.4 (Baxter, supplemented with about 2% human albumin). It is compatible with survival of the cells. It may comprise physiological levels of NaCl.

The pharmaceutical composition of the invention, which preferably comprises macrophages, may be comprised in a container suitable for spraying the pharmaceutical composition, e.g., with an atomizer.

The present invention thus also provides an application system comprising

a) a pharmaceutical composition comprising myeloid cells such as macrophages in a pharmaceutically acceptable carrier, and

b) a container suitable for spraying the pharmaceutical composition, e.g., with an atomizer.

Preferably, said macrophages are macrophages obtainable with the method of the invention, e.g., having the characteristics described herein. However, macrophages generated by other methods can also be employed in the application system of the invention, e.g., for the treatment as described herein.

Suitable containers are known in the art, e.g., from EP2743343A1. For example, RenovaCare's Skin Gun may be used (RenovaCare), which is capable of spraying a liquid suspension of cells, e.g., onto wounds. A gentle positive-pressure air stream may be used to spray the cell suspension. Such application systems are particularly useful for spraying the cell population of the invention, e.g., comprising macrophages, on surfaces, e.g., on wounds such as burns and/or infected wounds. They are also useful for treatment in the lung. They may also be used for treatment of a surface *in vitro,* e.g., for disinfection or for reducing the number of pathogens, in particular, for reducing the number of bacteria on said surface.

The invention also provides a pharmaceutical composition or the application system of the invention, preferably, comprising macrophages such as the macrophages obtainable by the method of the invention, for use in treating or preventing an infection in a patient, e.g., a human patient.

The inventors have shown that the pharmaceutical composition can advantageously be used for treatment or prevention of a bacterial infection, most preferably, for treatment of a bacterial infection. A bacterial infection may be, e.g., an infection with *P. aeruginosa, S. pneumoniae, S. aureus,* and *M. tuberculosis.* It may be a pulmonary infection. Treatment for pulmonary infections, e.g., with these bacteria, may be pulmonary e.g. by local cell administration via intubation. Alternatively, inhalation-based systems such as aerosol based sprays or systemic administration by i.v.

injections may be applied. Local administration may be used.

Bacterial pulmonary infections, e.g., with *P. aeruginosa,* are of particular relevance in patients such as cystic fibrosis patients. Patients for which the treatment or prevention may be of particular relevance are immunocompromised patients such as immunosuppressed patients, e.g., transplantation patients, patients in intensive care (e.g., with ventilation-associated pneumonia), newborn babies, elderly patients, patients suffering from chronic lung disease such as cystic fibrosis, CGD (or other genetic diseases leading to immunodeficiency), chronic obstructive pulmonary disease or hPAP.

Treatment of a bacterial infection may also be treatment of sepsis. In this context, macrophages can not only serve to reduce bacterial load, but the can also serve as a sink for reducing the load of pro-inflammatory cytokines, cell- and bacterial-debris, and/or toxins (macrophage sponge).

Treatment or prevention of an infection can also be treatment of an infected wound or prevention of infection of a wound. In that case, topical administration, e.g., by spraying the wound with an administration device of the invention is useful. The wound may be cleaned, e.g., removing coagulated blood, to facilitate access before treatment. Treatment or prevention of an infection can also be peri- or post-operative treatment with a composition of the invention, e.g., by spraying the composition of the invention on an operation site during the operation or after closing the operation site.

The invention also provides a pharmaceutical composition or the application system of the invention, preferably, comprising macrophages, for use in promoting wound healing in a patient. A wound may, e.g., be a burn, in particular, a grade II or grade III burn. Promoting wound healing may or may not be in the context of infection. Macrophages do not only contribute to elimination of pathogens, but they also assist in remodelling tissue. These characteristics make the pharmaceutical composition of the invention useful for treatment, e.g., of ulcers or for removal of necrotic tissue. Further uses may be e.g., in promoting transplantation success or in treatment of paradontitis. The composition may also be used to prevent or minimize scar formation.

In the context of the invention, treatment means that at least one symptom of a disease is ameliorated. Preferably, in the context of a bacterial infection, the bacterial load is also reduced, and most preferably, all bacteria are eliminated.

Prevention means that the patient to be treated is not yet ill, i.e., does not show symptoms of the disease, and may be not infected (yet). Prevention reduces the likelihood of occurrence of a disease (in particular, an infection) and/or reduces the severity of symptoms of a disease.

Typically, the invention will be used in the context of treatment, but prevention can also be of interest, e.g., in the context of an outbreak of multiple-resistant bacteria and/or with patients, e.g, immunocompromised patients, likely to be in contact with such bacteria, e.g., in intensive care or in the context of solid organ transplantation or blood/bone marrow transfusion.

One particular advantage of the invention is that it can be used to treat or prevent a bacterial infection with bacteria resistant to at least one antibiotic agent, or with multi-resistant bacteria.

Alternatively, treatment and/or prevention, preferably, treatment of viral infections, e.g., influenza (Cardani et al. 2017), with pharmaceutical compositions comprising cell populations of the invention (preferably, comprising macrophages) is contemplated. For treatment of influenza, e.g., intrapulmonary application, e.g., as described herein, or spray application, may be carried out. If the viral pathogen is known, antibodies (of a suitable species which can be recognized by the macrophages in the composition) directed to surface antigens of the virus may further be included in the pharmaceutical composition. Antibodies directed against surface antigens of bacteria can also be added to compositions intended for treatment of bacteria.

Typically, administration of the pharmaceutical composition will be at the site of an infection or at the site of a potential infection. For example, for pulmonary infection, pulmonary (intrapulmonary) administration is optimal. However, if the pulmonary disease is so severe that it is likely that the cells will not reach the site of infection and/or that an intubation would be of high risk to the patient, e.g., in the case of severe COPD or severe pulmonary infection, i.e., other modes of application are also contemplated. Other suitable modes of administration include i.v. administration, inhalation based systems (sprays, e.g., similar to an asthma spray), topical administration, e.g., to wounds such as infected wounds or wounds in danger of being infected, ocular administration (e.g., upon ocular infection), nasal administration, administration to an organ due to be transplanted or administration near the side of infection (e.g. near to implant associated infections).

In general, iPSC from the patient (e.g., a human patient) may be used for generation of the myeloid cells and, accordingly, autologous myeloid cells (preferably, macrophages) of the invention. However, this is not required, as the elimination of bacteria by allogeneic macrophages administered to a patient may be quicker than rejection of the macrophages. If long term survival of the transplanted macrophages is not required or maybe even not desirable, e.g., in acute bacterial infections without a genetic disease background, allogeneic macrophages, producible in a cost-effective way by industrial scale bioreactor systems as an off-the-shelf product, could be applied. In particular, in the context of pulmonary administration, survival and functionality of administered macrophages after administration has been shown.

Of note, the use of allogeneic myeloid cells of the invention would, at least in immunocompetent recipients, minimize the risk of tumor formation, since the allogeneic cells can be expected to be immunologically rejected within the first two weeks. Nevertheless, repeated application of off-the-shelf preparations would be possible, if different HLA-haplotypes are applied. It is also possible to use myeloid cells (e.g., macrophages) generated form pluripotent stem cells (e.g., iPSC) either selected (or engineered) for immunocompatibility or lack of expression of HLA molecules.

Based on the experiments conducted by the inventors, about $4 \times 10^6$ macrophages (calculated for total cell numbers in the cell population of the invention) successfully treated pulmonary infections in mice of about 25 g. Lower cell numbers are expected to be sufficient for treatment, e.g., about $4 \times 10^5$ to about $4 \times 10^6$ per mouse, or about $1 \times 10^6$ to about $2 \times 10^6$ per mouse. This translates to about $1,6 \times 10^7$/kg to about $1,6 \times 10^8$/kg or about $5 \times 10^7$/kg to $1 \times 10^8$/kg. Consequently, for example, a 60 kg human patient may be treated with about $1 \times 10^9$ to $1 \times 10^{10}$ macrophages. The number of cells required for successful treatment of course depends on a number of factors which can be assessed by the clinician, e.g., the general health of the patient, in particular, the immune status, the severity of the disease and bacterial load. It is considered that significantly less cells are needed for prevention than for treatment. In case of wound healing or wound infections, similar assessments by the clinician maybe performed to elucidate therapeutic cell numbers. For example, $4 \times 10^5$ to about $8 \times 10^6$ macrophages /cm$^2$ may be sufficient for treatment.

In addition to the application of iPSC-derived macrophages, e.g., for the treatment of infectious diseases, other iPSC-derivatives may alternatively or additionally be applied to target a variety of diseases. Suspension-based differentiation cultures also allow for the generation of other iPSC-derived myeloid cells, and the stirred tank bioreactor technology is also suitable for the generation, e.g., of iPSC-derived granulocytes. Such granulocytes may, e.g., be used in combination with macrophages and/or alone for an improved treatment of bacterial infections, e.g., as listed above, or for the treatment of fungal infections.

Thrombocytes obtainable according to the method of the invention may be suitable e.g., for use in the treatment of thrombocytopenia, in particular following anti-cancer chemotherapy and/or hematopoietic stem cell transplantation either prophylactically (in particular to prevent intracranial bleeding) preemptive (e.g. in anticipation of major surgery) or therapeutically in bleeding episodes. Similarly such thrombocytes could be used in functional platelet deficiencies such as drug- and here in particular aspirin-induced or congenital such as Glanzmann thrombasthenie or Bernard Soulier syndrome.

Dendritic cells obtainable according to the method of the invention may be suitable e.g. for use in vaccination strategies against (i) tumors and other malignancies or (ii) pathogens including viruses such as EBV or CMV.

Alternatively, the large-scale differentiation with only IL3 could be employed to generate a myeloid progenitor population suitable for cryopreservation and the subsequent on demand differentiation towards therapeutic cell types of choice.

Cell populations of the invention, preferably, comprising macrophages, are also useful for applications, e.g., *in vitro,* such as

a) drug screening and drug development;

b) disease modelling;

c) tissue engineering (e.g., blood production for trauma therapy);

d) preparation of bioartifical organisms;

e) disinfection and/or elimination of pathogens (e.g., for water purification or disinfection of surfaces);

f) coating of materials for transplantation, e.g., stents;

g) development of biomarkers, e.g., in haematopoietic differentiation, development and maturation of blood cells, preferably macrophages, to monitor physiology and pathophysiology of said cells, wherein, for example, expression profiles of cells from a patient and a healthy person (or a standard cell for comparison) may be screened; or

h) quality control of biological products selected from the group comprising antibodies, hormones, cytokines, drugs, culture medium, and serum.

**Figure legends**

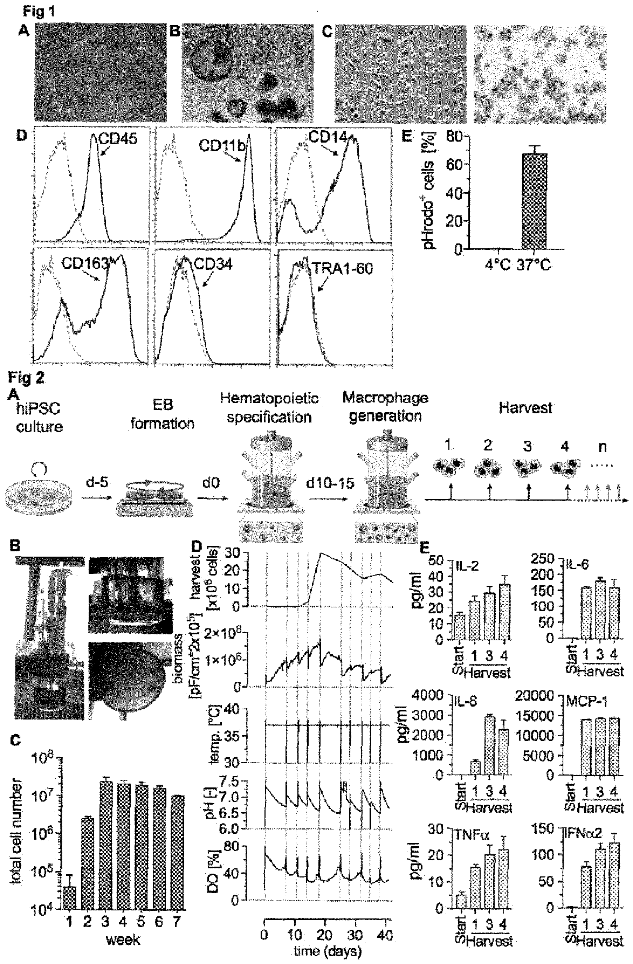

**Fig. 1 Hematopoietic differentiation of human iPSC in suspension culture.** (**A**) Representative brightfield image of undifferentiated human iPSC (CD34iPSC16). Scale bar, 500µm. (**B**) Representative brightfield image of CD34iPSC16 differentiated towards a myeloid cell forming complex (MCFC) producing iPSC-derived macrophages (iPSC-MACs). Scale bars, 500$\mu$m. (**C**) Brightfield microscopy (left) and Cytospin staining (right) of iPSC-MACs. Scale bars, 100$\mu$m. (**D**) IPSC-MACs analyzed by flow cytometry (dashed line: respective isotype control, black line: surface marker). (**E**) Phagocytosis of pHrodo-labeled *E.Coli* particles by iPSC-MACs at 4°C (negative control) or at 37°C (n=2, mean±s.e.m.). See also Figure S1.

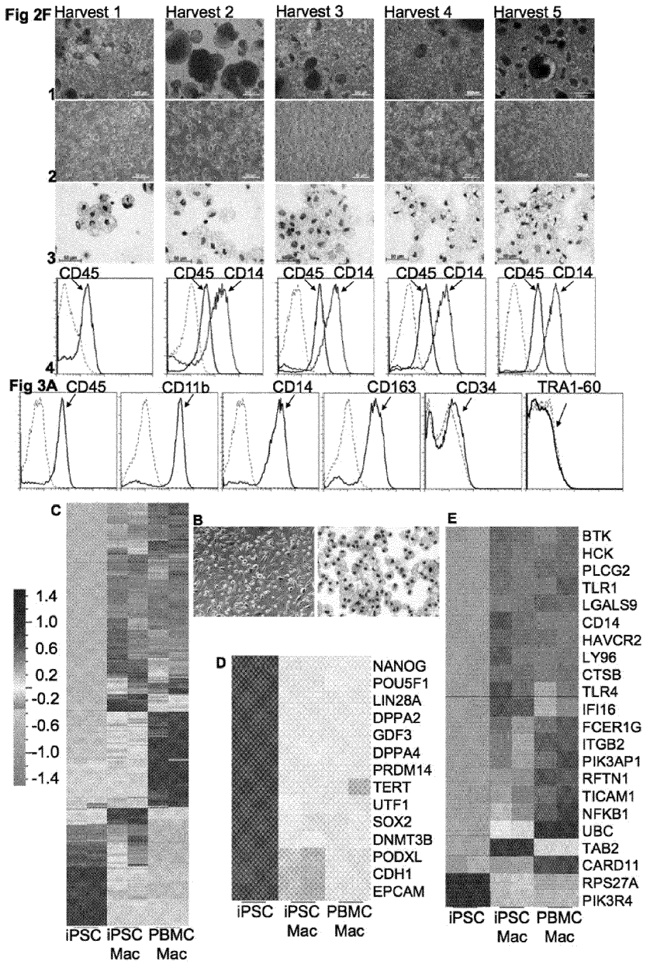

**Fig. 2 Continuous generation of human iPSC-MACs in stirred tank bioreactors.** (**A**) Scheme of hematopoietic differentiation of human iPSC in stirred tank bioreactors (DASbox system). (**B**) Representative pictures of DASbox bioreactor filled with floating MCFCs. (**C**) Individual cell counts of viable macrophages produced in bioreactors (n=2 of independent bioreactor runs, mean±s.e.m.). (**D**) Representative macrophage harvest counts (upper graph) and corresponding data from continuous process monitoring (biomass, temperature, pH and dissolved oxygen (DO) level) for the entire cultivation phase of a 42-day bioreactor run. (**E**) Analysis of human cytokines in the medium from the bioreactor (technical duplicates). (**F**) Representative light microscopy of macrophage generation analyzed at harvests 1-5. First row shows brightfield images of non-filtered medium samples from bioreactors. Second row shows brightfield images of freshly harvested macrophages separated from MCFCs by sedimentation. Third and fourth row show cytospin and flow cytometric analysis of iPSC-MACs (dashed line: respective isotype control, blackline: CD45, dotted line CD14). Scale bars, 50, 100 and 500$\mu$m, respectively. See also Figure S2.

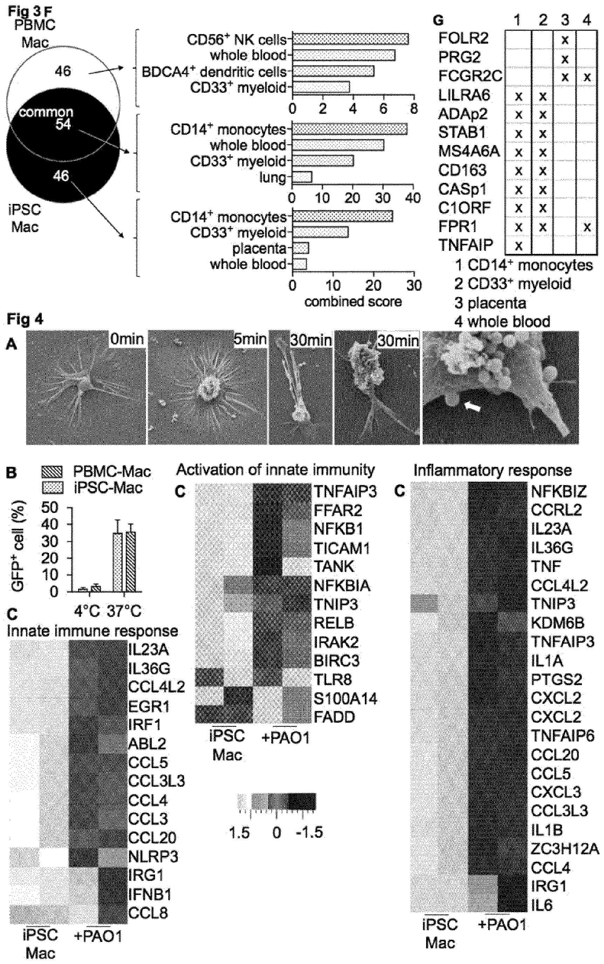

**Fig. 3 Characterization of iPSC-MACs derived from stirred tank bioreactors.** (**A**) Representative flow cytometric analysis of iPSC-MACs derived from bioreactor differentiation (dashed line: respective isotype control, black line: cell surface marker). (**B**) Representative pictures of either brightfield (left image) or cytospin preparations (right image) of terminally differentiated iPSC-MACs derived from the bioreactor. Scale bars, 200 and 100$\mu$m. (**C-E**) Transcriptome analysis of iPSC, iPSC-derived macrophages (iPSC-MACs) derived from bioreactors and PBMC-derived macrophages (PBMC-MACs) (n=2, biological replicates). (**C**) Unbiased hierarchical heatmap clustering. (**D**) Heatmap of pluripotency-associated genes. (**E**) Heatmap of differentially regulated genes (p<0.001) associated with activation of innate immune response (GO:0002253). (**F**) Venn-diagramm of upregulated genes in iPSC-MACs and PBMC-MACs (Top 100; compared to undifferentiated iPSC) and cell type assignment based on the upregulated genes for the commonly shared gene set as well as the gene set obtained for iPSC-MACs and PBMBC-Mac, respectively, according to the human gene atlas (EnrichR). (**G**) Clustergram depicting matched genes from the cell line assignment of the genes upregulated in iPSC-MAC only (EnrichR).

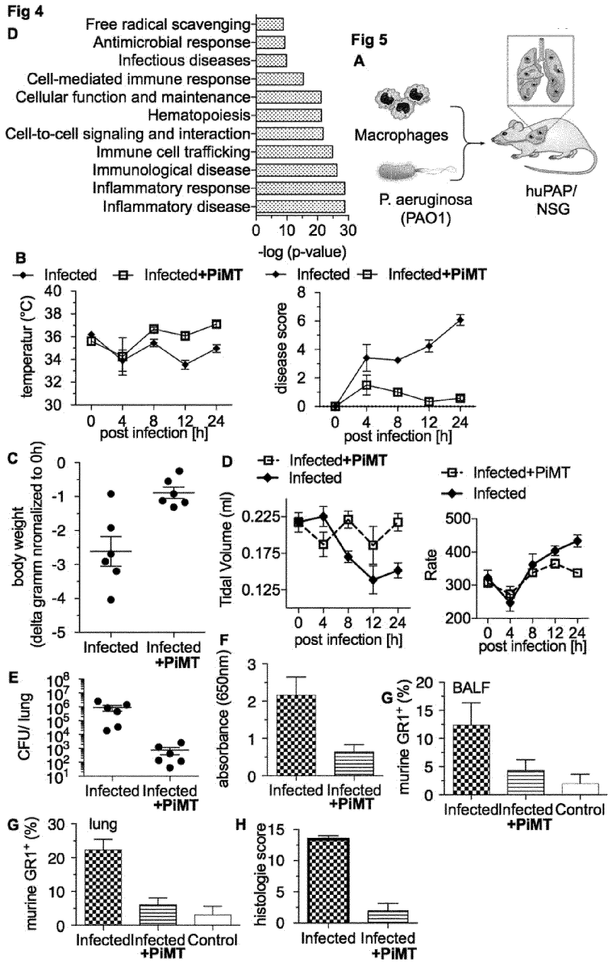

**Fig. 4 Antimicrobial activity of iPSC-MACs generated in stirred tank bioreactors.** (**A**) Scanning raster electron microscopy depicting phagocytosis of latex beads by iPSC-MACs at different time points (**B**) Rate of phagocytosis by terminally differentiated iPSC-MACs and PBMC-MACs after 2h of incubation with GFP-labeled *P. aeruginosa* (PAO1) at 4 or 37°C (n=3 of biological replicates, two-way ANOVA with Sidak's multiple comparisons test, ns denotes not significant). (**C**) Heatmap of differentially regulated genes associated with inflammatory response (GO:0006954, >10-fold upregulated), innate immune response (GO:0045087, >5-fold regulated) and activation of innate immune response (GO:0002253, >2-fold regulated). (**D**) IPA analysis for disease and function of >5-fold upregulated genes.

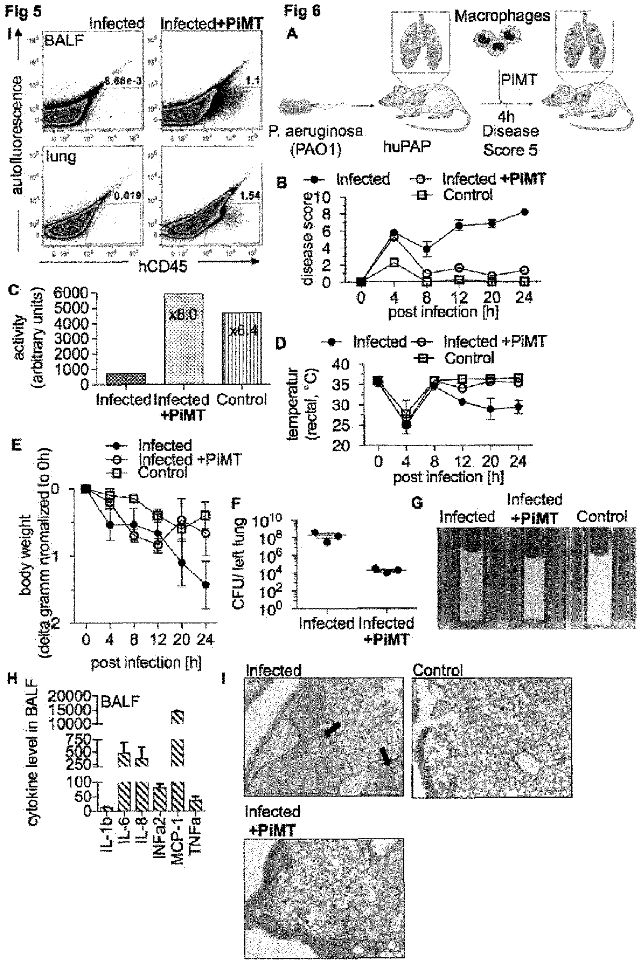

**Fig. 5 Pulmonary infection and simultaneous macrophage transplantation in huPAP mice.** (**A**) Scheme of pulmonary transfer of *P. aeruginosa* (PAO1) and simultaneous transplantation of iPSC-MACs (PiMT) into huPAP or NSG mice. (**B**) Time course of rectal temperature and disease score over 24h after infection of huPAP mice with PAO1 (infected) or infected and transplanted (infected+PiMT) (n=6 animals/group, mean±s.e.m). (**C**) Change in body weight after 24h. Values are normalized to the respective weights before infection (n=6 animals/group, mean±s.e.m). (**D**) Lung function measured by head-out bodyplethysmography (n=6 animals/group, mean±s.e.m). (**E**) Bacterial colony forming units (CFU) of PAO1 per lung after 24h (n=6 animals/group, mean±s.e.m). (**F**) Absorbance of bronchio-alveolar lavage fluid (BALF) samples at 650nm as a measure for the presence of hemoglobin (n=3 animals/group, mean±s.e.m). (**G**) Flow cytometric analysis of BALF and lung. Percentage of mouse granulocytes (determined as GR1[+] cells) in BALF and Lung (n= 3 animals/group, mean±s.e.m). (**H**) Right lung histological scoring (n=3 animals/group, mean±s.e.m). (**I**) Representative diagram of BALF and lung of one animal per group stained with hCD45.

**Fig. 6 Pulmonary infection and therapeutic transplantation of iPSC-MACs in huPAP mice.** (**A**) Scheme of pulmonary infection with *P. aeruginosa* (PAO1) and therapeutic transplantation of iPSC-MACs (PiMT) derived from bioreactors into huPAP mice (**B**) Time course of disease scores of huPAP mice infected with PAO1 (infected), infected and transplanted (infected+PiMT) and control mice receiving PBS twice (control) (n=3 animals/group, mean±s.e.m). (**C**) Activity 24h post-infection evaluated by movement of mice analyzed by video documentation and manual tracking (n=1 animal/group). (**D**) Time course of rectal temperature analyzed over 24h after infection (n=3 animals/group, mean±s.e.m). (**E**) Change in body weight after 24 hours. Values are normalized to the respective weights before infection (n=3 animals/group, mean±s.e.m). (**F**) Bacterial colony forming units (CFU) of PAO1 per lung after 24 hours (n=3 animals/group, mean±s.e.m). (**G**) Images of BALF samples. (**H**) Levels of human cytokines in BALF of infected+PiMT mice (n=2 mice). (**j**) Lung histology images of infected, infected+PiMT and control mice.

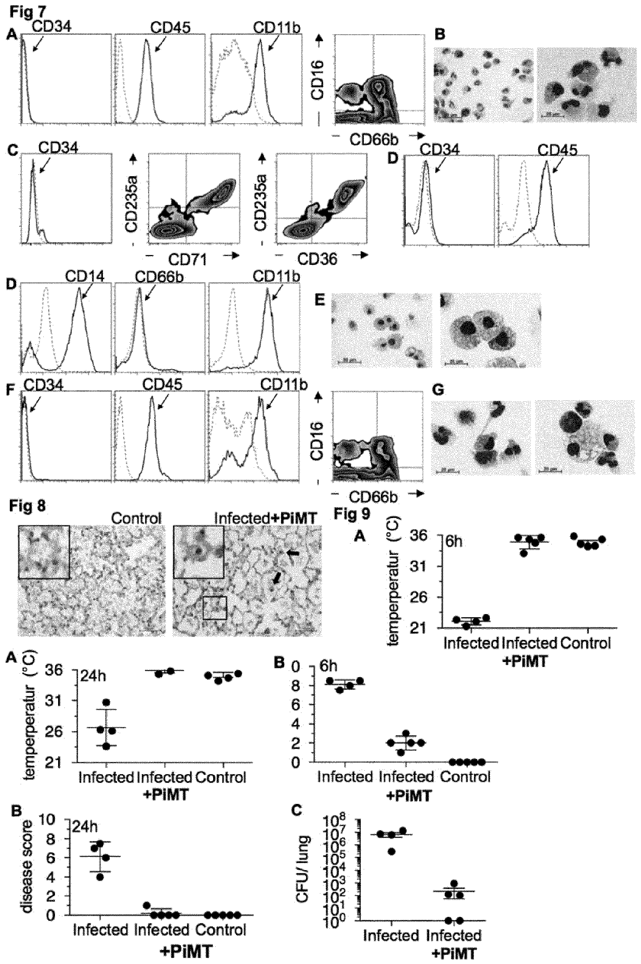

**Fig. 7 Derivation of myeloid cells from hematopoietic differentiation in suspension.** (**A**) Flow cytometric analysis of suspension-based hematopoietic differentiation cultures of cells generated by IL3 and G-CSF supplementation and finally differentiated in the presence of G-CSF only (dashed line: respective unstained control, black line: cell surface marker). (**B**) Cytospins of cells generated from IL3/G-CSF cultures, terminally differentiated by G-CSF only. Scale bar: 50μm and 20μm. (**C**) Flow cytometric analysis of suspension-based hematopoietic differentiation cultures of cells generated by IL3 and SCF/EPO supplementation and finally differentiated in the presence of SCF and EPO (dashed line: respective isotype control, black line: cell surface marker). (**D**) Flow cytometric analysis of cells derived from suspension cultures using IL3 only and differentiated for more than seven days in M-CSF. Data shown as histogram overlays (dashed line: respective unstained control, black line: cell surface marker). (**E**) Respective cytospin for IL3-derived cultures which have been terminally differentiated by M-CSF. Scale bar: 50μm and 20μm. (**F**) Flow cytometric analysis of cells derived from suspension cultures using IL3 only and differentiated for more than seven days in G-CSF. Data shown as histogram overlays (dashed line: respective unstained control, black line: cell surface marker. (**G**) Respective cytospin for IL3-derived cultures which have been terminally differentiated by G-CSF. Scale bar: 50μm and 20μm.

**Fig. 8 Lung histology of transplanted mice.** Right lung histology showing macrophages in infected+PiMT animals only (representative image, n=3). Scale bar, 50μm.

**Fig. 9 Pulmonary infection and simultaneous macrophage transplantation in NSG mouse models. (A)** Rectal temperature and (**B**) disease score of NSG mice infected with PAO1 (infected) or infected and transplanted (infected+PiMT) 6h and 24h post-infection. Disease Score and temperatures measured before the experiment served as control values (n= 4-5 animals/group, mean±s.e.m). (**C**) Colony forming units (CFU) of PAO1 per lung after 24h in infected and infected+PiMT NSG mice (n=4-5 animals/group, mean±s.e.m).

## References

Ackermann, M., et al. (2015). Lost in translation: pluripotent stem cell-derived hematopoiesis. EMBO molecular medicine 7, 1388-1402.

Aloush, V., et al. (2006). Multidrug-resistant Pseudomonas aeruginosa: risk factors and clinical impact. Antimicrobial agents and chemotherapy 50, 43-48.

Bjarnsholt, T., et al. (2005). Pseudomonas aeruginosa tolerance to tobramycin, hydrogen peroxide and polymorphonuclear leukocytes is quorum-sensing dependent. Microbiology 151, 373-383.

Bonfield, T.L., et al. (2012). Absence of the cystic fibrosis transmembrane regulator (Cftr) from myeloid-derived cells slows resolution of inflammation and infection. J Leukoc Biol 92, 1111-1122.

Bruscia, E.M., et al. (2009). Macrophages directly contribute to the exaggerated inflammatory response in cystic fibrosis transmembrane conductance regulator-/- mice. Am J Respir Cell Mol Biol 40, 295-304.

Buchrieser, J., et al. (2017). Human Induced Pluripotent Stem Cell-Derived Macrophages Share Ontogeny with MYB-Independent Tissue-Resident Macrophages. Stem cell reports 8, 334-345.

Cardani A. et al., (2017). Alveolar Macrophages prevent lethal influenza pneumonia by inhibiting infection of type-1 alveolar epithelial cells. PLoS Pathog 13(1): e1006140.

Chen, K.G., et al. (2014). Human pluripotent stem cell culture: considerations for maintenance, expansion, and therapeutics. Cell stem cell 14, 13-26.

Choi, K.D., et al. (2009). Generation of mature human myelomonocytic cells through expansion and differentiation of pluripotent stem cell-derived lin-CD34+CD43+CD45+ progenitors. The Journal of clinical investigation 119, 2818-2829.

Dias, J., et al. (2011). Generation of red blood cells from human induced pluripotent stem cells. Stem cells and development 20, 1639-1647.

Dutow, P., et al. (2013). Severity of allergic airway disease due to house dust mite allergen is not increased after clinical recovery of lung infection with Chlamydia pneumoniae in mice. Infection and immunity 81, 3366-3374.

Falgenhauer, et al. (2016). Colistin resistance gene mcr-1 in extended-spectrum beta-lactamase-producing and carbapenemase-producing Gram-negative bacteria in Germany. Lancet Infect Dis 16, 282-283.

Feng, Q., et al. (2014). Scalable generation of universal platelets from human induced pluripotent stem cells. Stem cell reports 3, 817-831.

Freire-Moran, et al. (2011). Critical shortage of new antibiotics in development against multidrug-resistant bacteria-Time to react is now. Drug Resist Updat 14, 118-124.

Gordon, S.B., and Read, R.C. (2002). Macrophage defences against respiratory tract infections. Br Med Bull 61, 45-61.

Gould, I.M. (2013). Treatment of bacteraemia: meticillin-resistant Staphylococcus aureus (MRSA) to vancomycin-resistant S. aureus (VRSA). Int J Antimicrob Agents 42 Suppl, S17-21.

Happle, C., et al. (2014). Pulmonary transplantation of macrophage progenitors as effective and long-lasting therapy for hereditary pulmonary alveolar proteinosis. Science translational medicine 6, 250ra113.

Hauser, A.R., et al. (2011). Clinical significance of microbial infection and adaptation in cystic fibrosis. Clin Microbiol Rev 24, 29-70.

Herigstad, B., et al. (2001). How to optimize the drop plate method for enumerating bacteria. J Microbiol Methods 44, 121-129.

Hirsch, E.B., and Tam, V.H. (2010). Impact of multidrug-resistant Pseudomonas aeruginosa infection on patient outcomes. Expert Rev Pharmacoecon Outcomes Res 10, 441-451.

Kempf, H., et al. (2016). Large-scale production of human pluripotent stem cell derived cardiomyocytes. Adv Drug Deliv Rev 96, 18-30.

Kempf, H., et al. (2016b). Bulk cell density and Wnt/TGFbeta signalling regulate mesendodermal patterning of human pluripotent stem cells. Nature communications 7, 13602.

Kempf, H., et al. (2015). Cardiac differentiation of human pluripotent stem cells in scalable suspension culture. Nature protocols 10, 1345-1361.

Kempf, H., et al. (2014). Controlling expansion and cardiomyogenic differentiation of human pluripotent stem cells in scalable suspension culture. Stem cell reports 3, 1132-1146.

Klockgether, et al. (2010). Genome diversity of Pseudomonas aeruginosa PAO1 laboratory strains. J Bacteriol 192, 1113-1121.

Kropp, C., et al. (2016a). Impact of Feeding Strategies on the Scalable Expansion of Human Pluripotent Stem Cells in Single-Use Stirred Tank Bioreactors. Stem cells translational medicine 5, 1289-1301.

Kropp, C., et al. (2016b). Progress and challenges in large-scale expansion of human pluripotent stem cells. Process Biochemistry.

Lachmann, N., et al. (2015). Large-scale hematopoietic differentiation of human induced pluripotent stem cells provides granulocytes or macrophages for cell replacement therapies. Stem cell reports 4, 282-296.

Lachmann, N., et al. (2014). Gene correction of human induced pluripotent stem cells repairs the cellular phenotype in pulmonary alveolar proteinosis. American journal of respiratory and critical care medicine 189, 167-182.

Lavin, Y., et al. (2014). Tissue-resident macrophage enhancer landscapes are shaped by the local microenvironment. Cell 159, 1312-1326.

Liu, Y.Y., et al. (2016). Emergence of plasmid-mediated colistin resistance mechanism MCR-1 in animals and human beings in China: a microbiological and molecular biological study. Lancet Infect Dis 16, 161-168.

Ma, F., et al. (2008). Generation of functional erythrocytes from human embryonic stem cell-derived definitive hematopoiesis. Proceedings of the National Academy of Sciences of the United States of America 105, 13087-13092.

Mizgerd, J.P. (2008). Respiratory infection and the impact of pulmonary immunity on lung health and disease. Am J Respir Crit Care Med 186, 824-829.

Munder, A., et al. (2005). Murine pulmonary infection with Listeria monocytogenes: differential susceptibility of BALB/c, C57BL/6 and DBA/2 mice. Microbes Infect 7, 600-611.

Ng, E.S., et al. (2008). A protocol describing the use of a recombinant protein-based, animal product-free medium (APEL) for human embryonic stem cell differentiation as spin embryoid bodies. Nature protocols 3, 768-776.

Oliver, A., et al. (2000). High frequency of hypermutable Pseudomonas aeruginosa in cystic fibrosis lung infection. Science 288, 1251-1254.

Oliver, A., et al. (2015). The increasing threat of Pseudomonas aeruginosa high-risk clones. Drug Resist Updat 21-22, 41-59.

Olmer, R., et al. (2012). Suspension culture of human pluripotent stem cells in controlled, stirred bioreactors. Tissue Eng Part C Methods 18, 772-784.

Pasula, R., et al. (2016). Airway delivery of interferon-gamma overexpressing macrophages confers resistance to Mycobacterium avium infection in SCID mice. Physiol Rep 4.

Pineros, A.R. et al. (2017). M2 macrophages or IL-33 treatment attenuate ongoing Mycobacterium tuberculosis infection. Scientific Reports 7, Article number 41240.

Rakhimova, E., et al. (2009). Pseudomonas aeruginosa population biology in chronic obstructive pulmonary disease. The Journal of infectious diseases 200, 1928-1935.

Ruehl-Fehlert, C., et al. (2003). Revised guides for organ sampling and trimming in rats and mice--part 1. Exp Toxicol Pathol 55, 91-106.

Schroeder, M.R., and Stephens, D.S. (2016). Macrolide Resistance in Streptococcus pneumoniae. Front Cell Infect Microbiol 6, 98.

Shi, C., and Pamer, E.G. (2011). Monocyte recruitment during infection and inflammation. Nature reviews Immunology 11, 762-774.

Sturgeon, C.M., et al. (2014). Wnt signaling controls the specification of definitive and primitive hematopoiesis from human pluripotent stem cells. Nature biotechnology 32, 554-561.

Suzuki, T., et al. (2014). Pulmonary macrophage transplantation therapy. Nature 514, 450-454.

Trapnell, B.C., et al. (2003). Pulmonary alveolar proteinosis. The New England journal of medicine 349, 2527-2539.

van de Laar, L., et al. (2016). Yolk Sac Macrophages, Fetal Liver, and Adult Monocytes Can Colonize an Empty Niche and Develop into Functional Tissue-Resident Macrophages. Immunity 44, 755-768.

van Wilgenburg, B., et al. (2013). Efficient, long term production of monocyte-derived macrophages from human pluripotent stem cells under partly-defined and fully-defined conditions. PloS one 8, e71098.

Ventola, C.L. (2015). The antibiotic resistance crisis: part 1: causes and threats. P T 40, 277-283.

Weiss, G., and Schaible, U.E. (2015). Macrophage defense mechanisms against intracellular bacteria. Immunological reviews 264, 182-203.

Willinger, T., et al. (2011). Human IL-3/GM-CSF knock-in mice support human alveolar macrophage development and human immune responses in the lung. Proceedings of the National Academy of Sciences of the United States of America 108, 2390-2395.

Willyard, C. (2017). The drug-resistant bacteria that pose the greatest health threats. Nature 543, 15.

Wolbeling, F., et al. (2010). Head-out spirometry accurately monitors the course of Pseudomonas aeruginosa lung infection in mice. Respiration 80, 340-346.

Zhang, H., et al. (2015). Functional analysis and transcriptomic profiling of iPSC-derived macrophages and their application in modeling Mendelian disease. Circulation research 117, 17-28.

Zweigerdt, R. (2009). Large scale production of stem cells and their derivatives. Adv Biochem Eng Biotechnol 114, 201-235.

Zweigerdt, R., et al. (2011). Scalable expansion of human pluripotent stem cells in suspension culture. Nature protocols 6, 689-700.

WO2010/025776A1, WO 2010/099539A1, WO 2016/114723A1, WO 2016/127259A1, EP2743343A1

## Examples

## Methods

## Cell Culture and Differentiation

### iPSC cultivation

Human iPSC (hCD34iPSC16) have been previously generated from mobilized peripheral blood CD34[+] cells(Lachmann et al., 2014) and were culture on irradiated murine embryonic fibroblasts (MEF) in iPSC medium (knockout DMEM, 20% knock out serum replacement, 1mM L-glutamine, 1% NEAA, 1% penicillin/streptomycin (all Invitrogen, Karlsruhe, Germany), 0.1mM β-mercaptoethanol (Sigma-Aldrich, St. Louis, MO, United States), and 10ng/ml bFGF (PeproTech). Basic-FGF was omitted from the maintenance medium for the last 4-5 days prior to EB formation.

### Hematopoietic differentiation in adherent culture

Classical adherence-based hematopoietic differentiation of human iPSC was performed as previously described (Lachmann et al., 2015). In brief, PSC colonies (3 wells of a 6-well plate, approx. $3\times10^6$ cells) were disrupted to fragments using collagenase-IV, and EB formation was induced by cultivation for 5 days in iPSC medium supplemented with $10\mu$M Rock inhibitor (Y-27632; Tocris) in six-well suspension plates on an orbital shaker. Subsequently, EBs were manually selected using a binocular and transferred to tissue culture six-well plates and cultivation in differentiation medium I (X-VIVO 15, Lonza) supplemented with 1% penicillin-streptomycin (Life Technologies), 25ng/ml human IL3 and 50ng/ml human M-CSF (both PeproTech). From day 10-15 onwards, iPSC-MACs were harvested from the supernatant once a week.

## Hematopoietic differentiation in suspension culture

To achieve hematopoietic differentiation in suspension, largest EBs were selected by sedimentation properties (sedimentation <10 min) and transferred to differentiation medium I in 6-well suspension plates on the orbital shaker (Celltron, Infors HT) at 85 revolutions per minute (rpm). From day 10-15 onwards, iPSC-MACs were harvested from the medium once a week. For differentiation into other myeloid cell types, APEL medium was used as described previously (Ng et al., 2008). For differentiation into granulocytic and erythroid cells, 25ng/ml hIL3 combined with 50ng/ml hG-CSF or 100ng/ml hSCF plus 3U hEPO, respectively, were applied.

## Hematopoietic differentiation in stirred tank bioreactors

The bioreactor (DASbox Mini bioreactor system, Eppendorf) was setup and calibrated as previously described(Kempf et al., 2015). In brief, the 250ml glass vessel was equipped with an 8-blade impeller (60° pitch) and probes for online monitoring of biomass (Aber Instruments), pH, DO as well as control of temperature. Calibration was performed in 120ml chemically defined X-VIVO 15 (Lonza).

For hematopoietic differentiation in the bioreactor, iPSC were expanded to twenty 6-well plates and cultivated for 3 days in the presence of bFGF. EB formation was performed equivalent to adherent cultures. After 5 days, EBs were selected by sedimentation properties (sedimentation <10 min) and transferred to the equilibrated bioreactor. Cells were cultivated in X-VIVO 15 supplemented with IL3 and M-CSF (differentiation medium I) at 37°C with constant headspace-gassing at 3L/h (21% $O_2$; 5% $CO_2$) and stirring at 50rpm. To monitor MCFCs integrity and macrophages formation, 1ml samples were collected 1-2 times per week via the sampling port without interrupting the culture process. Differentiation medium I was manually replaced every 6-7 days with optional fed of 20ml after 3-4 days. Macrophages were harvested weekly by separation from MCFC via sedimentation (4-5 min) and subsequent filtering of the medium through a 100μm strainer. Retained MCFCs were returned to the bioreactor. Macrophages were collected from filtered medium via centrifugation at 300xg for 4min.

Data from online monitoring were processed using Microsoft Excel 2016 and GraphPad Prism 6. Supernatant was analysed for concentration of glucose and lactate using YSI 2700 select biochemistry analyser, for osmolarity using Osmomat 300 (Gonotec) and for concentration of lactate dehydrogenase according to manufacturer's instruction (MAK066, Sigma) using a microplate reader (Paradigm, Beckman Coulter).

## Terminal differentiation

For further maturation, cells freshly harvested from MCFCs, were cultured in differentiation medium II (RPMI1640 medium supplemented with 10% fetal calf serum (FCS), 2mM L-glutamine, 1% penicillin-streptomycin) containing 50ng/ml hM-CSF for macrophage, 50ng/ml hG-CSF for granulocyte, and 100ng/ml SCF and 3U/ml EPO for erythroid differentiation for at least 7 days.

## Isolation of peripheral blood mononuclear cells (PBMCs) and differentiation to macrophages

All healthy donors gave written informed consent according to the local ethical committee at Hannover Medical School. Peripheral blood mononuclear cells (PBMC) were isolated from the peripheral blood of healthy volunteers by gradient centrifugation using Biocoll Separating Solution (40 min, 400xg; Biochrome, Billerica, MA). Subsequently, cells were cultured in RPMI1640 medium supplemented with 10% fetal calf serum, 2mM L-glutamine, 1% penicillin-streptomycin (all Invitrogen), and hIL3 and hM-CSF (50ng/ml each, PeproTech) for one week. After this, PBMC-MACs were cultivated for further 3-4 days in differentiation medium containing 50ng/ml M-CSF only.

## Phenotypic and Functional Characterization

## Flow cytometry

Flow cytometric analysis of myeloid cells was performed as described(Lachmann et al., 2015; Lachmann et al., 2014). For macrophages, PBS supplemented with 10% FCS was used to prevent unspecific binding. Cells were analyzed with a FACScalibur cytometer (Beckton & Dickinson, Heidelberg, Germany) and analyzed with FlowJo software (TreeStar, Ashland, OR). Antibodies were purchased from eBioscience: hTRA-1-60-PE (Cat-No: 12-8863-80), hCD11b-APC (Cat-No: 17-0118-41), hCD14-PE (Cat-No: 12-0149-42), hCD163-APC (Cat-No: 17-1639-41, hCD16-FITC (Cat-No: 11-0168-41), hCD34-FITC (Cat-No: 11-0349-41) and isotype-controls: mouse-IgG1a-PE (Cat-No: 12-4714-41), FITC (Cat-No: 11-4714-41) or APC (Cat-No: 17-4714-41), and rat-IgG2a-PE (Cat-No: 12-4321-81). Antibodies from Biolegend San Diego, CA, United States: hCD86-APC (Cat-No: 305411), hCD66b-FITC (Cat-No: 305104) or hCD45-PE (Cat-No: 304007).

For flow-cytometric analysis of mouse lung and BALF, samples were fixed using 4%PFA. After this, samples were incubated with fc receptor blocking antibodies (CD16/CD32, eBioscience, Cat-No: 14-0161-81) for 20 min to prevent unspecific binding. Used antibodies were purchased from Biolegend San Diego, CA, United States: hCD45-PeCy7, and eBioscience: mGR1-eFluor450.

## Cytospin preparation

20,000-50,000 cells were spun on glass slides at $600 \times g$ for 7 min and stained for 5 min in 0.25% May-Grünwald and 20 min in 0,4% Giemsa stain modified solution (Sigma).

## Phagocytosis Assays

The phagocytic activity of iPSC-MACs derived from suspension or adherent cultures and terminally differentiated on tissue-culture plates was assessed by flow cytometry. Therefore, $1 \times 10^5$ cells were incubated with pHrodo™ Red *E. codi* BioParticles® Conjugate (MolecularProbes/ Thermo Fisher Scientific, Schwerte, Germany) or medium for 2h at 37° or 4°C as a negative control. After incubation the cells were put on ice for 10min. Analysis was performed using a Beckman Coulter FC500 flow cytometer.

The functional capacity of iPSC-MACs and PBMC-MACs to phagocytose vital *P. aeruginosa* was assessed using GFP-PAO1 (wild-type *P. aeruginosa* PAO1 tagged with green fluorescent protein (GFP) by Tn7 transformation (Bjarnsholt et al., 2005), (kindly provided by Thomas Bjarnsholt, University of Copenhagen) and compared to PBMC-derived Macrophages (PBMC-MACs). For the phagocytosis assay, $1 \times 10^5$ iPSC-MACs were incubated for 2 h with $6 \times 10^5$ CFU GFP-PAO1 at 37 °C or 4 °C as a negative control. Medium controls were treated similar. After incubation, cells were put on ice for 10 min and subsequently fixed with 2% paraformaldehyde (PFA) solution for 30 min. Analysis was performed using a Beckman Coulter FC500 flow cytometer.

## Electron microscopy

Macrophages were grown on 1cm diameter round coverslips. Latex beads ($1 \mu m$ diameter) were added to cells at 4°C and adhesion of latex beads to the surface of the macrophages was allowed for 5 min. Following samples were washed with cold PBS and warmed up with fresh culture medium to 37°C. Phagocytosis was allowed for up to 1h and samples were fixed at different time points using 1.5% Paraformaldehyde, 1.5% Glutaraldehyde in 150mM Hepes pH 7.35. Samples were then dehydrated using an increasing methanol series. Critical point drying was performed using a CPD030 critical point dryer (Balzers, Lichtenstein) following manufacturer instructions. Coverslips were then sputtered with gold (Sem Coating System, Polarion) and SEM was carried out using a Philips SEM 505 (Eindhoven, The Netherlands).

## Bacterial culture

For experiments *P. aeruginosa* laboratory strain *PAO1 (Klockgether et al., 2010)* was taken from a stock culture kept at -80°C and grown in Luria Broth (LB) overnight. After washing with sterile PBS the desired infectious dose was extrapolated from a standard growth curve. For the determination of the actual dosage, inoculates were serially plated on LB agar plates via the drop-plate method (Herigstad et al., 2001) and CFU determined after 16-18h incubation at 37°C.

## Collection of microarray samples

Terminally differentiated human iPSC-MACs or human PBMC-MACs were seeded on 24-well plates (500.000cells/well) and cultured overnight. On the next day, cells were washed three times with PBS. Subsequently, *P. aeruginosa* laboratory strain PAO1 in RPMI medium without antibiotics (MOI10) was centrifuged onto the cells (600xg) and incubated at 37°C. Cells with medium only served as non-infected controls. After 1h cells were de-attached, washed and resuspended in RNA lysis buffer. RNA isolation was performed with RNAeasy micro Kit (Quiagen) according to manufacturer's instructions. Human iPSC samples were obtained after sorting of iPSC for TRA-1-60+ to separate iPSC from feeder cells.

## Microarray experiments (Single Colour Mode)

The Microarray utilized in this study represents a refined version of the Whole Human Genome Oligo Microarray 4x44K v2 (Design ID 026652, Agilent Technologies), called '054261On1M' (Design ID 066335) developed in the Research Core Unit Transcriptomics (RCUT) of Hannover Medical School. Microarray design was created at Agilent's eArray portal using a 1x1M design format for mRNA expression as template. All non-control probes of design ID 026652 have been printed five times within a region comprising a total of 181560 Features (170 columns x 1068 rows). Four of such regions were placed within one 1M region giving rise to four microarray fields per slide to be hybridized individually (Customer Specified Feature Layout). Control probes required for proper Feature Extraction software operation were determined and placed automatically by eArray using recommended default settings.

30ng of total RNA were used to prepare aminoallyl-UTP-modified (aaUTP) cRNA (Amino Allyl MessageAmp™ II Kit; #AM1753; Life Technologies) as directed by the company (applying one-round of amplification). The labelling of aaUTP-cRNA was performed by use of Alexa Fluor 555 Reactive Dye (#A32756; LifeTechnologies).

cRNA fragmentation, hybridization and washing steps were carried-out as recommended in the 'One-Color Microarray-Based Gene Expression Analysis Protocol V5.7', except that 500ng of each fluorescently labelled cRNA population were used for hybridization.

Slides were scanned on the Agilent Micro Array Scanner G2565CA (pixel resolution 3 $\mu$m, bit depth 20). Data extraction was performed with the 'Feature Extraction Software V10.7.3.1' using the extraction protocol file 'GE1_107_Sep09.xml', except that 'Multiplicative detrending' algorithm was inactivated.

Measurements of on-chip replicates (quintuplicates) were averaged using the geometric mean of processed intensity values of the green channel, 'gProcessedSignal' (gPS) to retrieve one resulting value per unique non-control probe. Single Features were excluded from averaging, if they i) were manually flagged, ii) were identified as Outliers by the Feature Extraction Software, iii) lie outside the interval of '1.42 x interquartile range' regarding the normalized gPS distribution of the respective on-chip replicate population, or iv) showed a coefficient of variation of pixel intensities per Feature that exceeded 0.5.

Averaged gPS values were normalized by quantile normalization approach first. Subsequently, values were additionally processed by global linear scaling: All gPS values of one sample were multiplied by an array-specific scaling factor. This factor was calculated by dividing a 'reference 75th Percentile value' (set as 1500 for the whole series) by the 75th Percentile value of the particular Microarray to be normalized ('Array I' in the formula shown below). Accordingly, normalized gPS values for all samples (microarray data sets) were calculated by the following formula:

$$normalized \; gPS_{Array \; i} = gPS_{Array \; i} \; x \; (1500 \, / \, 75^{th} \; Percentile_{Array \; i})$$

Finally, a lower intensity threshold (surrogate value) was defined based on intensity distribution of negative control features. This value was fixed at 15 normalized gPS units. All of those measurements that fell below this intensity cutoff were substituted by the respective surrogate value of 15.

Normalized microarray data of all non-control features were imported into Omics Explorer software v3.2 (Qlucore) under default import settings for Agilent One Color mRNA Microarrays, except that any normalization option was deselected. Accordingly, data processing steps during import were: 1) log base 2 transformation, 2) baseline transformation to the median.

Heatmap clustering analysis and generation of GO-based heatmaps were performed in Omics Explorer. Top 100 upregulated genes were calculated using the RCUTAS tool (V1.7; Hannover Medical School) and processed using Venny 2.1 (http://bioinfogp.cnb.csic.es/tools/venny). Gene set enrichment analysis for cell type classification based on the human gene atlas and gene ontology analysis of biological processes and molecular function were conducted using Enrichr (https://amp.pharm.mssm.edu/Enrichr). Gene analysis for disease and function were performed using Ingenuity pathway analysis (Qiagen).

Microarray data were deposited under accession number E-MTAB-5436 in the ArrayExpress database (www.ebi.ac.uk/arrayexpress).

**Cytokine secretion assays (Luminex)**

In order to analyze the secretion of human cytokines in bioreactor samples or BALF samples, Luminex® analysis with a Cytokine Human 14-Plex Panel (Millipore, Schwalbach, Germany) was performed as described before (Lachmann et al., 2015). Data were acquired on a Luminex-200 System and analyzed with the Xponent software v.3.0 (Life Technologies).

**In vivo experiments**

**Animal maintenance and infection**

HuPAP (129S4-Rag2$^{tm1.1Flv}$ Csf2/Il3$^{tm1.1(CSF2,IL3)Flv}$ Il2rg$^{tm1.1Flv}$/J) mice (Willinger et al., 2011) were obtained from the Jackson Laboratory and housed in the central animal facility of Hannover Medical School. NSG mice (NOD.Cg-*Prkdc*<sup>*scid*</sup> *Il2rg*<sup>*tmMjl*</sup>/SzJZtm) were obtained from the central animal facility of Hannover Medical School. Both immunodeficient mouse strains were maintained under pathogen free conditions in individually ventilated cages (IVC) with free access to food and water. All animal experiments were approved by the Lower Saxony State animal welfare committee and performed according to their guidelines.

For infection with *P. aeruginosa* or pulmonary iPSC-MAC transplantation, anesthetized (ketamine/midazolam) mice were instilled via the trachea after oral intubation. For the simultaneous infection experiments, iPSC-MAC ($4x10^6$/animal) and PAO1 (NSG: $5x10^5$ and huPAP $0.2x10^5$ CFU) were resuspended in PBS and mixed in a total volume of $60\mu l$. For solely infected mice the same CFU was apllied in $60\mu l$ PBS. To avoid phagocytosis before instillation, cell/bacteria mixes were kept on ice all the time. For the therapeutic PiMT experiments, huPAP animals were infected with $0.3x10^5$ CFU PAO1 (in a volume of $30\mu l$ PBS) after anesthesia with ketamine/midazolam. Control mice received the same volume PBS. After 4h, mice were anesthetized by isoflurane inhalation and the second instillation with $50\mu l$ PBS or $4x10^6$ iPSC-MACs in PBS was performed. Control mice again received the same volume PBS. To determine the disease score of the animals we used a scoring matrix as described previously (Munder et al., 2005). After 24h, animals were sacrificed and end-analysis was performed.

### Murine lung function

Non-invasive head-out spirometry investigating 14 lung function parameters was performed on conscious restrained mice as previously described(Wolbeling et al., 2010). Mice were positioned in glass inserts, their breathing causes air to flow through a pneumotachograph. A pressure transducer creates an electrical signal, which is analysed using NOTOCORD HEM software (Version 4.2.0.241, Notocord Systems SAS, Croissy Sur Seine, France). The parameters of tidal volume (measured in ml), expiratory time, inspiratory time, time of inspiration+expiration, relaxation time and the flow at 50% of the expiratory tidal volume (EF50) were selected to characterize murine lung function during infection.

### Broncho-aleveolar lavage (BAL) and measurement of hemoglobin levels

Broncho-alveolar lavage was performed by cannulating the murine trachea post-mortem. The right lung was rinsed with 1ml of PBS for three times. Fresh BALF was used for photometric analysis of haemoglobin. After this, BALF samples were centrifuged and supernatants were stored at -80°C for Luminex analysis. Pellets were fixed and stained for flow cytometry

### Lung bacterial numbers (CFU)

The right lungs of the euthanized mice were ligated, resected and homogenized with a tissue homogenizer (Polytron PT 1200, Germany). Total bacterial numbers were assessed from serial dilutions of the homogenates which were cultured on Luria-Bertani plates using the drop plate method (Herigstad et al., 2001).

### Histology

At the dedicated time points the animals were sacrificed. Right lungs were filled with OCT buffer and fixed in neutral buffered 4% PFA for 3 days at 4°C. For control animals, left lungs were used. Tissues were trimmed according to the RITA-Guidelines (Ruehl-Fehlert et al., 2003), dehydrated (Shandon Hypercenter, XP) and subsequently embedded in paraffin (TES, Medite). Sections (2-3 $\mu$m thick, microtom Reichert-Jung 2030) were deparaffinized in xylene and H&E stained according to standard protocols. Blinded evaluation (Axioskop 40, Zeiss microscope) and histological scoring of the sections was performed as described before (Dutow et al., 2013) by a trained pathologist.

### Statistics

GraphPad Prism 6 and 7 was applied to perform unpaired Student's T test or analysis of variance (ANOVA). Unless otherwise stated, mean$\pm$s.e.m. is plotted. Asterisks denote: * $P < 0.05$; ** $P < 0.01$; *** $P < 0.001$; **** $P < 0.0001$.

### *Results*

### Derivation of multiple human iPSC-derived myeloid lineages in dynamic suspension culture

Although the generation of different mature hematopoietic cell types from PSC has been proven successful using classical two-dimensional (2D) differentiation cultures (Ackermann et al., 2015; Choi et al., 2009; Dias et al., 2011; Feng et al., 2014; Sturgeon et al., 2014), these systems do not allow for the generation of iPSC-derived cells in clinically relevant quantities. Thus, the inventors developed a suspension-based (3D) hematopoietic differentiation protocol, suitable for process upscaling in industry-compatible stirred tank bioreactors (Kropp et al., 2016b; Zweigerdt, 2009). Using a well-characterized hiPSC line (hCD34iPSC16) (Lachmann et al., 2014) with classical brightfield morphology (**Fig**. **1A**), they first induced the formation of embryoid bodies (EBs) in small-scale suspension culture on an orbital shaker. After 5 days, they transferred the EBs to differentiation medium containing IL3 and M-CSF to induce hematopoietic specification and the formation of myeloid cell forming complexes (MCFCs) (**Fig**. **1B**). After 10-15 days of continued suspension culture, MCFCs continuously produced iPSC-derived macrophages (iPSC-MACs) (4D-differentiation) that could be harvested weekly for up to three months. Generated iPSC-MACs displayed typical macrophage-like morphology, exhibited a highly pure surface marker profile of $CD45^+CD11b^+CD14^+CD163^+CD34^-TRA1-60^-$, and efficiently phagocytosed fluorescently labeled *E. coli* particles (**Fig. 1C-1E**).

Of note, different myeloid subsets were generated in following the same suspension-based protocol but simply switching the cytokine composition. Thus, IL3 and G-CSF allowed continuous generation of $CD16^+CD66b^+$ iPSC-granulocytes (**Fig. 7A and 7B**), whereas $CD34^-CD71^+CD235a^+CD36^+$ erythroid cells were derived by the use of IL3/SCF/EPO (**Fig. 7C**). In contrast to cells generated by IL3 in combination with lineage instructive cytokines, employing IL3 only led to display of a more immature $CD45^+/CD11b^+/CD14^-/CD163^-$ marker profile (data not shown). Of note, further differentiation of these immature cells in the presence of either M-CSF or G-CSF led to the generation of $CD45^+CD11b^+CD14^+CD66b^-$ $CD34^-$ macrophage-like (**Fig. 7D and 7E**) or $CD16^+CD66b^+$, granulocyte-like cells (in the following also designated macrophages and granulocytes) (**Fig. 7F and 7G**).

**Up-scaling continuous production of hiPSC-MACs is enabled in stirred tank bioreactors**

The inventors then translated the suspension-based differentiation into stirred tank bioreactors using an industry-compatible system (DASbox Mini Bioreactor System)(Olmer et al., 2012) previously applied for the efficient cultivation of human iPSC and their differentiation into cardiomyocytes (Kempf et al., 2014) (**Fig. 2A, 2B**). Bioreactors were equipped with probes for real-time monitoring of dissolved oxygen (DO), pH, temperature and impedance-based biomass assessment. Process parameters were set to 37°C, headspace-gassing at 3L/h (21% $O_2$; 5% $CO_2$) and stirring at 50 revolutions per minute (rpm) using an 8 blade-pitched (60°) impeller (Kempf et al., 2015). With respect to future clinical scale-up, it is noteworthy that a chemically defined culture medium (X-Vivo15) which is also applied in clinical trials, was used. From day 7-10 onwards, weekly harvest of iPSC-MACs from the uninterrupted bioreactor process showed an increase in cell yield over time, reaching stable production of approx. 2-3x10$^7$ iPSC-MACs/week as early as week three, which was maintained for more than five weeks in two independent process runs (**Fig. 2C**). The efficient generation of iPSC-MACs in both bioreactor experiments was reflected by the weekly increase in biomass, particularly during the first days after full medium refreshment. DO and pH monitoring revealed expected zigzag-like patterns typical for repeated batch cultures. The pH was ranging from 7.25 (fresh medium) down to 6.5, resulting from typical medium acidification by the cells' metabolic activity, particularly by lactate release (Kropp et al.). Notably, all process parameters showed maintenance of repetitive patterns after reaching the steady state of macrophage production around d15-20, confirming the overall stability of the process (**Fig. 2D**). This finding is further supported by stable values for glucose, lactate, lactate dehydrogenase, and osmolality, which were determined weekly in parallel to macrophage harvests. Similar, secretion of cytokines/chemokines associated with the activation of macrophages, such as IL2, IL6, IL8, MCP1, TNF$\alpha$, and IFN$\alpha$2, was detected from the first harvest (week 2) onwards (**Fig. 2E**) and corresponded to the appearance of $CD45^+$ iPSC-MACs. Notably, MCFCs cultivated in the bioreactor sustained their morphology during the entire process and continuously generated iPSC-MACs of typical morphology and a $CD45^+CD14^+$ surface marker profile, which increased in purity over time (**Fig. 2F**). Moreover, the extended cultivation of MCFCs derived from the bioreactor in 6-well suspension plates on an orbital shaker resulted in continued production of iPSC-MACs for additional 8 weeks (data not shown).

**Bioreactor-derived iPSC-MACs recapitulate phenotypic and transcriptional characterization of PBMC-MACs**

Detailed characterization of bioreactor-derived iPSC-MACs revealed a $CD45^+CD11b^+CD163^+CD14^+CD34^-TRA1-60^-$ phenotype, and a typical morphology after adherence to tissue culture plates (**Fig. 3A and 3B**). Comparison of iPSC-MACs to non-differentiated hiPSC and Peripheral Blood Mononuclear Cells (PBMC)-derived macrophages (PBMC-MACs) by unsupervised, hierarchical heatmap clustering of whole transcriptomes revealed close proximity of iPSC-MACs and PBMC-MACs when compared to iPSC (**Fig. 3C**). Analysis of genes associated with pluripotency and activation of innate immune response confirmed efficient differentiation of iPSC into macrophage-like cells (**Fig. 3D and 3E**). Importantly, genes associated with macrophage function such as the toll-like receptors (TLR) 1 and 4, CD14, or components of the NF-$\kappa$B signaling pathway (gene ontology (GO) Activation of innate immunity: 0002218) were significantly upregulated in iPSC-MACs and PBMC-MACs versus iPSCs (**Fig. 3E**).

Comparison of the top 100 upregulated genes in iPSC-MACs versus pluripotent hiPSC and PBMC-MACs versus pluripotent hiPSC revealed a common gene set of 54 upregulated genes. These transcripts including *CD14, CD68, CSFR1, CCR1* and *CYBB* are assigned to CD14$^+$ monocytes, whole blood and CD33$^+$ myeloid cells (**Fig. 3F**). Of note, genes of this set exclusively upregulated in iPSC-MACs (46 genes) were also assigned to CD14$^+$ monocytes and CD33+ myeloid cells and included genes such as *CD163*, the leukocyte immunoglobulin like receptor A6 (*LILPA6*), stabilin 1 (STAB1) or the formyl peptide receptor 1 (*FPR1*) (**Fig. 3G**). In contrast, 46 genes of this set upregulated in PBMC-MACs only revealed the highest score in gene sets associated with CD56$^+$ natural killer (NK) cells, whole blood and dendritic cells (e.g. human leukocyte antigens (*HLA*), *CCL5/RANTES* or granzyme (*GZM*) A and B), an observation maybe explained by contaminating CD56$^+$/CD3$^-$ NK-cells (data not shown). Table 1 shows the top 100 genes which are differentially regulated (either upregulated or downregulated) in the macrophages of the invention directly compared to macrophages derived from PBMC.

*Table 1*

| *Characteristic gene expression pattern* of *iPS-Mac (compared to PBMC-Mac)* ||
|---|---|
| **upregulated** | **downregulated** |
| DKK1 | ANPEP |
| SEPP1 | CDA |
| PITX2 | CRTAM |
| COL3A1 | ENST00000390237 |
| KRT19 | FBP1 |
| A_33_P3221980 | GBP5 |
| CALD1 | GNLY |
| CYR61 | HLA-DQA1 |
| H19 | HLA-DQA2 |
| DDIT4L | HLA-DQB1 |
| FRZB | HLA-DQB2 |
| TMEM98 | HLA-DRA |
| NNMT | HLA-DRB1 |
| NPNT | HLA-DRB3 |
| LUM | HLA-DRB4 |
| DCN | HLA-DRB5 |
| LYVE1 | IL15 |
| MGP | LY75 |
| IGFBP3 | S1PR4 |
| NUAK1 | TNFAIP6 |

***In vitro* antimicrobial activity of bioreactor-derived iPSC-MACs**

Next, bioreactor-derived iPSC-MACs were evaluated for their *in vitro* antimicrobial activity. Scanning electron microscopy (SEM) at different time points after incubation of iPSC-MACs with fluorescently labeled latex beads revealed typical changes in the overall cell morphology early after the stimulus and efficient phagocytic uptake of beads over time (**Fig. 4A**). Even more important, iPSC-MACs also phagocytosed GFP-labeled *P. aeruginosa* at 37°C in comparable efficiency to PBMC-MACs, whereas no phagocytosis was observed at 4°C, confirming active phagocytosis (**Fig. 4B**). To gain insights into the ability of iPSC-MACs to remodel their transcriptome towards the signature of activated macrophages, whole transcriptome analysis of iPSC-MACs was performed before and after contact with *P. aeruginosa* (PAO1). Hierarchical cluster analysis of gene ontologies (GOs) associated with inflammatory response or (activation of) innate immune response showed marked upregulation of cytokines (e.g. *IL23A, TNF*α, *IL1A, IL6, INFG1*), chemokines (e.g. *CCL5, CCL20, CCL4, CXCL3*) and molecules involved in NFκB signaling in response to pathogen contact (**Fig. 4C**). Similarly, gene ontology enrichment analysis of >5 fold upregulated genes after pathogen contact revealed high scores for GOs associated with biological processes, including inflammatory response, response to lipopolysaccharide (LPS) and molecules of bacterial origin as well as response to wounding. Moreover, GOs associated with molecular function revealed enrichment of GO terms such as cytokine/chemokine activity and receptor binding or G-protein coupled receptor binding. Enrichment analyses of these upregulated genes for disease and function revealed ontologies including inflammatory disease and response, immune cell trafficking, antimicrobial response and free radical scavenging (**Fig. 4D**).

**iPSC-MACs prevent respiratory infections by *Pseudomonas aeruginosa***

To evaluate the therapeutic efficacy of iPSC-MACs *in vivo,* a humanized mouse model C;129S4-Rag2[tm1.1Flv] Csf2/Il3[tm1.1(CSF2,IL3)Flv] Il-12rg[tm1.1Flv]/J (huPAP mice), an immunodeficient strain with impaired alveolar macrophage development (Willinger et al., 2011) was utilized. HuPAP mice recapitulate hallmarks of the human disease pulmonary alveolar proteinosis (PAP), including the susceptibility to pulmonary infections. In addition, the iPSC-MACs were utilized in a second immunodeficient mouse model: NOD.Cg-*Prkdc^scid Il2rg^{tm1Wjl}*/SzJZtm (NSG), which have impaired lymphopoiesis, but show normal alveolar macrophage development.

First, huPAP mice were infected with the *P. aeruginosa* laboratory strain PAO1 and co-administered with $4\times10^6$ iPSC-MACs in the same instillation process (solely *PAO1* infected mice served as controls). The infection course was closely monitored and mice were sacrificed 24h post-infection for end analysis (**Fig. 5A**). Infected huPAP mice displayed clinical symptoms already 4h post-infection as indicated by an elevated disease score of 3.5 $\pm$0.9, which gradually increased over time up to 6.1$\pm$0.4 after 24h. In addition, a decrease in rectal temperature to 35.0$\pm$0.3°C and body weight loss of 2.6$\pm$0.5g was observed in infected animals 24h post-infection (all mean$\pm$s.e.m., n=6) (**Fig. 5B and 5C**). In contrast, animals receiving a simultaneous pulmonary iPSC-MACs transplantation (PiMT) showed only mild symptoms of infection (**Fig. 5B**). This was in line with normal rectal temperature of 37.1$\pm$0.2°C, a very low disease score of 0.6$\pm$0.2 and only little loss of body weight of - 0.89$\pm$0.2g 24h post-infection in PiMT treated animals (all mean$\pm$s.e.m., n=6) (**Fig. 5B and 5C**). Similar beneficial effects of PiMT were demonstrated by head-out bodyplethysmography to measure lung function (Wolbeling et al., 2010). While infected animals showed a decrease in tidal volume as well as expiratory and inspiratory time and an increase in breathing rate, animals from the infected+PiMT group showed normal values for all parameters analyzed (**Fig. 5D**). As a consequence of PiMT, transplanted mice showed significantly reduced bacterial numbers in the lung 24h post-infection compared to their non-transplanted controls (**Fig. 5E**). Moreover, erythrocytes were present only in broncho-alveolar lavage fluid (BALF) from infected mice not receiving PiMT (**Fig. 5F**). Lung inflammation was furthermore indicated by an increase in murine GR1[+] granulocytes in the BALF and lungs of infected animals, but not infected+PiMT or control mice (**Fig. 5G**). In addition, histological evaluation revealed massive granulocyte infiltration, severe hemorrhage and alveolar edema in infected mice (score: 13.7$\pm$0.3), whereas lungs from infected+PiMT animals showed merely slight changes (score: 2.0$\pm$1.2, both mean$\pm$s.e.m., n=3) (**Fig. 5H**). Reduced inflammation in infected+PiMT animals was associated with the detection of hCD45[+] cells in lung and BALF as well as presence of macrophages in histological sections of the right lung (**Fig. 5I and 8**).

Similar results were obtained in NSG mice, a second immunodeficient mouse strain with normal alveolar macrophage development. Infected NSG animals developed profound symptoms of infection, such as a high disease score and a profound drop in rectal temperature 6h and 24h post-infection. In clear contrast, animals simultaneously receiving iPSC-MACs displayed normal body temperature and only minimally increased disease scores 6h and 24h post-infection, respectively (**Fig. 9A and 9B**). Moreover, analysis of bacterial numbers 24h post-infection revealed a significantly reduced bacterial load in the lungs of infected+PiMT mice (**Fig. 9C**).

**Therapeutic PiMT rescues mice from severe respiratory infections**

After demonstrating the efficacy of iPSC-MACs in simultaneous infection experiments, the inventors evaluated a clinically more relevant therapeutic PiMT treatment approach. In these experiments, huPAP mice were infected intrapulmonary with *P. aeruginosa* and closely monitored for 3-4h until first disease symptoms developed (determined by a disease score $\geq$5). Subsequently, in this model, mice received $4\times10^6$ iPSC-MACs (infected+PiMT) only after the infection-related symptoms were readily manifested. Of note, infected control mice received PBS only instead of PiMT (infected) (**Fig. 6A**).

In this infection and treatment schedule, therapeutic PiMT treated mice showed a decrease in the disease score as well as a normalization of rectal temperature and body weight to a degree comparable to non-infected animals already within 4-8 hours post treatment. In contrast, infected mice receiving PBS showed pronounced disease progression over time (**Fig. 6B, 6D and 6E**). Of note, 24h post-infection, infected mice showed marked disease symptoms, whereas animals that received a therapeutic PiMT showed a significantly reduced disease score (1.8$\pm$0.2 infected+PiMT vs 8.1$\pm$0.2 for infected animals, mean$\pm$s.e.m., n=3). In addition, the elevated disease score in infected mice was associated with clearly restricted activity when compared to control animals and mice receiving the therapeutic PiMT (**Fig. 6C**). Efficiency of therapeutic PiMT was further documented by normalized rectal temperature and body weight values 24h post-infection, and a profound reduction in lung bacterial numbers in infected+PiMT mice (**Fig. 6B, 6D, 6F**). Recapitulating our findings in the simultaneous transplant model, BALF of animals from the infected+PiMT group showed reduced erythrocyte levels compared to infected, non-transplanted controls. This observation was accompanied by the detection of important pro-inflammatory human cytokines/chemokines such as hIL6, hILB, hINFa2, hMCP-1 and TNF$\alpha$ (**Fig. 6G and 6H**). Inflammation was furthermore assessed by lung histology sections. Here, infected mice showed several areas of massive granulocytic infiltration, severe hemorrhage and alveolar edema, which were barely detectable in mice treated with iPSC-MACs from the bioreactor (**Fig. 6I**).

*Discussion*

In the present study, the inventors have evaluated a treatment concept that utilizes macrophages as a cellular therapeutic for bacterial infections. Since clinically-relevant numbers of autologous or donor derived macrophages can hardly be produced from somatic sources, the inventors investigated the possibility to utilize hPSCs for generating substantial quantities of functional macrophages.

The use of iPSC derivatives as a cellular approach to target infections has not been seriously considered so far. This might be explained by the fact that the supposed potential of hPSC for the production of specific progenies in therapeutically relevant numbers has not yet been fully translated into practice. Taking advantage of recent advances in culture medium formulations, the inventors tried to culture and differentiate human PSCs as floating aggregates, and adapted stirred tank bioreactor technology to stem cell requirements, allowing for clinical scale-up of iPSC and their derivatives. In order to develop the application of iPSC- macrophages as a cellular therapeutic against bacterial infections, and more specifically to clear pulmonary infections triggered by *P. aeruginosa,* they first established a scalable and continuous hematopoietic differentiation process for human iPSC in fully equipped bioreactors.

For *in vivo* studies, huPAP mice were employed, an immunodeficient strain which lacks alveolar macrophages. This is of clinical relevance, as this mouse model recapitulates important hallmarks of pulmonary alveolar proteinosis (PAP) (Willinger et al., 2011), including the susceptibility to pulmonary infections typically observed in PAP patients (Trapnell et al., 2003). In this mouse model, prevention of acute *P. aeruginosa* infection by simultaneous PiMT, or, even more important, therapeutic PiMT was highly effective within a short time frame. These proof-of-concept experiments were performed with a high cell dose. Similar therapeutic effects may be achievable with lower cell numbers. However, even with this maximum cell dose, no apparent adverse events were observed in either treatment scenario, which concurs with observations from pulmonary macrophage transplantation therapy studies employing bone marrow-derived macrophages (BMDM) (Happle et al., 2014; Suzuki et al., 2014).

Considering a body weight of 25g per mouse, clinical translation of iPSC-MACs for the treatment of respiratory infections would require approximately $1 \times 10^{10}$ iPSC-MACs for a 60 kg patient. Even without further process optimization, this would translate to a 40-60L production scale, which is in principle feasible with current bioreactor technologies (Kropp et al., 2016b). Of note, continuous process monitoring of the bioreactor-based differentiation of the invention revealed a substantial increase in biomass especially in the first 2-3 days of the repeated batch culture. This was followed by partial recovery of dissolved oxygen levels and a profound drop in pH in the last days before medium refreshment. These observations suggest the presence of process-limiting factors and highlight the potential for further process optimization. This can be achieved by cultivation at higher cell densities combined with the application of perfusion systems and feedback control of oxygen, pH and other process parameters, which can multiple the increases in cell yields of hPSCs and their progenies (Kropp et al., 2016a).

Given the impressive *in vivo* functionality of iPSC-MACs in the acute infection model, a therapeutic efficacy of PiMT in chronic infections with *P. aeruginosa,* as frequently observed in patients suffering from chronic obstructive pulmonary disease (COPD) (Rakhimova et al., 2009) or cystic fibrosis (CF) (Oliver et al., 2000) is expected. Especially CF represents an interesting disease scenario for the application of PiMT, as the disease-causing mutations in the cystic fibrosis conductance regulator (CFTR) gene also hamper functionality of professional phagocytes and thus the host's defense against infections (Bonfield et al., 2012; Bruscia et al., 2009). While single PiMT showed therapeutic efficacy for acute infections, repetitive therapeutic intervention or the application of genetically enhanced cells (Pasula et al., 2016) might be required to clear the established infection in chronic diseases.

Long term engraftment and sustained functionality of the transplanted iPSC-MACS are expected. In the tested transplantation scenarios, iPSC-MACs were still detected at 24h, however later time point were not investigated in this study. Earlier studies demonstrated engraftment of BMDM for more than one year (Suzuki et al., 2014). Moreover, pulmonary transplantation of murine macrophages derived from different multipotent progenitor cells showed the capacity for chromatin remodeling, adaption to the local tissue environment and long-term integration (Happle et al., 2014; Lavin et al., 2014; Suzuki et al., 2014; van de Laar et al., 2016). Along this line, the iPSC-MACs of the invention demonstrate rapid upregulation of pro-inflammatory gene expression after pathogen contact *in vitro* as well as efficient anti-microbial activity *in vivo,* suggesting their ability to response to the inflammatory environment in the lung after transplantation.

The strong and rapid anti-microbial and therapeutic effect observed by the inventors is of particular importance for a broad applicability of iPSC-MACs to target bacterial infections and a quick clinical implementation. The anti-microbial activity of iPSC-MAC against *P. aeruginosa* was evaluated, however, a phagocyte-based cell therapy might allow for a broad spectrum of applications in a number of different infection scenarios caused by other gram negative or positive bacteria such as *Streptococcus pneumoniae* or *Staphylococcus aureus,* or pathogens associated with implant infections that meanwhile pose an immense health and economical problem. This being said, new form of treatment are of specific clinical relevance considering the increasing numbers of pathogens resistant to standard or reserve antibiotic therapy (Aloush et al., 2006; Falgenhauer et al., 2016; Gould, 2013; Hirsch and Tam, 2010; Liu et al., 2016; Schroeder and Stephens, 2016).

In summary, the inventors provide the therapeutic application of PSC-derived phagocytes for the treatment of bacterial infections. They demonstrate the feasibility of phagocyte production under defined conditions that allow for clinical and industrial scale up and provide evidence for the efficacy and safety of iPSC-MAC transplantation as a new treatment approach targeting, e.g., severe respiratory infections. This technology, including process upscaling and the generation of additional hematopoietic cell types as well as assessment in other preclinical models allow for innovative cell-based treatment strategies for a wide variety of diseases.

**Claims**

1. A method of producing myeloid cells, comprising steps of

    a) cultivating embryoid bodies in suspension culture in the presence of IL-3 and, optionally, at least one additional cytokine, for a sufficient period of time to produce myeloid cell forming complexes;
    b) cultivating the myeloid cell forming complexes in the presence of IL-3 and, optionally, at least one additional cytokine, in suspension culture for a sufficient period of time to produce myeloid cells; and
    c) isolating the myeloid cells.

2. The method of claim 1, wherein the embryoid bodies are derived from pluripotent stem cells, preferably induced pluripotent stem cells.

3. The method of any of the preceding claims, wherein the embryoid bodies are obtained by a method comprising cultivating pluripotent stem cells in suspension culture for a sufficient period of time to produce embryoid bodies.

4. The method of any of the preceding claims, wherein the suspension culture is carried out in a bioreactor allowing suspension culture selected from the group comprising a stirred tank bioreactor, Erlenmeyer flask, spinner flask, wave bioreactor and rotating wall bioreactor, preferably, a stirred tank bioreactor.

5. The method of any of the preceding claims, wherein the cells are human cells.

6. The method of any of the preceding claims, wherein

    a) the additional cytokine is M-CSF and the produced myeloid cells are macrophages; or
    b) the additional cytokine is G-CSF and the produced myeloid cells are granulocytes; or
    c) the additional cytokine is GM-CSF and the produced myeloid cells are macrophages and granulocytes; or
    d) the additional cytokines are SCF and EPO and the produced myeloid cells are erythroid cells; or
    e) the additional cytokines are SCF and TPO and the produced myeloid cells are megakaryocytes and/or thrombocytes; or
    f) the additional cytokine is GM-CSF and IL-4 and the produced myeloid cells are dendritic cells; or
    g) there is no additional cytokine to IL-3, and the produced myeloid cells are immature cells capable of further differentiation, wherein, optionally, said method further comprises

        i) cultivating said immature cells in the presence of M-CSF until macrophages are obtained; or
        ii) cultivating said immature cells in the presence of G-CSF until granulocytes are obtained;
        iii) cultivating said immature cells in the presence of GM-CSF until granulocytes and macrophages are obtained;
        iv) cultivating said immature cells in the presence of SCF and EPO until erythroid cells are obtained;
        v) cultivating said immature cells in the presence of SCF and TPO until megakaryocytes and/or thrombocytes are obtained;

    wherein, preferably, the additional cytokine is M-CSF and the produced myeloid cells are macrophages.

7. The method of any of the preceding claims, wherein the method allows for a continuous, preferably, a batch-continuous production of said myeloid cells.

8. The method of any of the preceding claims, wherein isolation comprises purifying the produced myeloid cells, preferably, the macrophages, to a purity of at least 50%.

9. A suspension culture comprising myeloid cell forming complexes which continuously produce myeloid cells and, optionally, myeloid cells, wherein, optionally, said suspension culture is obtainable from the method of claim 1b.

10. A cell population obtainable by the method of any of claims 1-8.

11. A cell population comprising CD45+CD11b+/CD14+/CD163+/CD34-TRA1-60-macrophages,
wherein, preferably, expression of at least 9 genes selected from a group consisting of DKK1, SEPP1, PITX2, COL3A1, KRT19, A_33_P3221980, CALD1, CYR61, H19, DDIT4L, FRZB, TMEM98, NNMT, NPNT, LUM, DCN, LYVE1, MGP, IGFBP3 and NUAK1 is at least 20fold upregulated in said macrophages compared to macrophages derived from PBMC;
wherein, optionally, said cell population is obtainable by the method of any of claims 1-8.

12. A cell population comprising immature CD45+CD11b+/CD14-/CD163- myeloid cells, wherein, optionally, said cell population is obtainable by the method of any of claims 1-8.

13. A pharmaceutical composition comprising a cell population of any of claims 10 to 12 in a pharmaceutically acceptable carrier, wherein the cell population preferably is the population of claim 11.

14. An application system comprising

   a) a pharmaceutical composition comprising myeloid cells, preferably, macrophages selected from the group comprising the cell populations of any of claims 10 to 12, in a pharmaceutically acceptable carrier, and
   b) a container suitable for spraying the pharmaceutical composition.

15. The pharmaceutical composition of claim 13 or the application system of claim 14 for use in treating or preventing an infection in a patient and/or for promoting wound healing,
wherein the pharmaceutical composition preferably is for treatment or prevention of a bacterial infection, most preferably, for treatment of a bacterial infection.

16. Use of the cell population of any of claims 10 to 12, for

   a) drug screening and/or drug development;
   b) disease modelling;
   c) tissue engineering;
   d) preparation of bioartifical organisms;
   e) disinfection;
   f) coating of materials for transplantation;
   g) development of biomarkers; or
   h) quality control of biological products selected from the group comprising antibodies, hormones, cytokines, drugs, culture medium, and serum;

   wherein the cell population preferably is the population of claim 11.

Fig 1

Fig 2

Fig 2F

Fig 3A

**Fig 3 F**

**Fig 4**

Fig 4
D

Fig 5
A

Free radical scavenging
Antimicrobial response
Infectious diseases
Cell-mediated immune response
Cellular function and maintenance
Hematopoiesis
Cell-to-cell signaling and interaction
Immune cell trafficking
Immunological disease
Inflammatory response
Inflammatory disease

-log (p-value)

Macrophages

P. aeruginosa
(PAO1)

huPAP/
NSG

B

Infected   Infected+PiMT   Infected   Infected+PiMT

C

D   Infected+PiMT   Infected

Infected+PiMT   Infected

E

F

G   BALF

G   lung

H

Fig 5

Fig 6

Fig 7

Fig 8

Fig 9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | NICO LACHMANN ET AL: "Large-Scale Hematopoietic Differentiation of Human Induced Pluripotent Stem Cells Provides Granulocytes or Macrophages for Cell Replacement Therapies", STEM CELL REPORTS, vol. 4, no. 2, 1 February 2015 (2015-02-01), pages 282-296, XP055384440, United States ISSN: 2213-6711, DOI: 10.1016/j.stemcr.2015.01.005 | 9-16 | INV. C12N5/078 C12N5/0787 C12N5/0786 A61K35/15 |
| A | * abstract * * figure 1A * | 1-8 | |
| X | Wai Feng Lim ET AL: "R Hematopoietic cell differentiation from embryonic and induced pluripotent stem cells", , 18 June 2013 (2013-06-18), XP055384447, Retrieved from the Internet: URL:https://www.ncbi.nlm.nih.gov/pmc/articles/PMC3706875/pdf/scrt222.pdf [retrieved on 2017-06-23] * abstract * * figure 3 * | 10,12-16 | |

TECHNICAL FIELDS
SEARCHED       (IPC)

C12N
A61K

-/--

The present search report has been drawn up for all claims

| Place of search | Date of completion of the search | Examiner |
|---|---|---|
| Munich | 27 June 2017 | Loubradou-Bourges, N |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

# EUROPEAN SEARCH REPORT

Application Number

EP 17 16 9454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOSSEINIZAND HASTI ET AL: "Agitation increases expansion of cord blood hematopoietic cells and promotes their differentiation into myeloid lineage", CYTOTECHNOLOGY, KLUWER ACADEMIC PUBLISHERS, DORDRECHT, NL, vol. 68, no. 4, 12 August 2015 (2015-08-12), pages 969-978, XP036014190, ISSN: 0920-9069, DOI: 10.1007/S10616-015-9851-3 [retrieved on 2015-08-12] * abstract * | 9,10, 12-16 | |
| X | WO 2013/094988 A1 (MOGAM BIOTECH RES INST [KR]; GREEN CROSS LAB CELL CORP [KR]) 27 June 2013 (2013-06-27) | 9,10, 13-16 | |
| A | * claims 1-18 * * paragraph [0092] * | 1-8 | |
| X | COLLINS P C ET AL: "Stirred culture of peripheral and cord blood hematopoietic cells offers advantages over traditional static systems for clinically relevant applications", BIOTECHNOLOGY AND BIOENGINEERING, WILEY ETC, vol. 59, no. 5, 5 September 1998 (1998-09-05), pages 534-543, XP009194741, ISSN: 0006-3592 | 9,10, 13-16 | TECHNICAL FIELDS SEARCHED (IPC) |
| A | * abstract * | 1-8 | |

The present search report has been drawn up for all claims

| Place of search | Date of completion of the search | Examiner |
|---|---|---|
| Munich | 27 June 2017 | Loubradou-Bourges, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 9454

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2017

| Patent document cited in search report | Publication date | Patent family member(s) | | Publication date |
|---|---|---|---|---|
| WO 2013094988 A1 | 27-06-2013 | AU | 2012354438 A1 | 10-07-2014 |
| | | CN | 104204194 A | 10-12-2014 |
| | | EP | 2794859 A1 | 29-10-2014 |
| | | JP | 6010136 B2 | 19-10-2016 |
| | | JP | 2015502756 A | 29-01-2015 |
| | | KR | 20140123503 A | 22-10-2014 |
| | | US | 2015118207 A1 | 30-04-2015 |
| | | WO | 2013094988 A1 | 27-06-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010025776 A1
- EP 2743343 A1
- WO 2010099539 A1
- WO 2016114723 A1
- WO 2016127259 A1

### Non-patent literature cited in the description

- **ACKERMANN, M. et al.** Lost in translation: pluripotent stem cell-derived hematopoiesis. *EMBO molecular medicine,* 2015, vol. 7, 1388-1402
- **ALOUSH, V. et al.** Multidrug-resistant Pseudomonas aeruginosa: risk factors and clinical impact. *Antimicrobial agents and chemotherapy,* 2006, vol. 50, 43-48
- **BJARNSHOLT, T. et al.** Pseudomonas aeruginosa tolerance to tobramycin, hydrogen peroxide and polymorphonuclear leukocytes is quorum-sensing dependent. *Microbiology,* 2005, vol. 151, 373-383
- **BONFIELD, T.L. et al.** Absence of the cystic fibrosis transmembrane regulator (Cftr) from myeloid-derived cells slows resolution of inflammation and infection. *J Leukoc Biol,* 2012, vol. 92, 1111-1122
- **BRUSCIA, E.M. et al.** Macrophages directly contribute to the exaggerated inflammatory response in cystic fibrosis transmembrane conductance regulator-/- mice. *Am J Respir Cell Mol Biol,* 2009, vol. 40, 295-304
- **BUCHRIESER, J. et al.** Human Induced Pluripotent Stem Cell-Derived Macrophages Share Ontogeny with MYB-Independent Tissue-Resident Macrophages. *Stem cell reports,* 2017, vol. 8, 334-345
- **CARDANI A. et al.** Alveolar Macrophages prevent lethal influenza pneumonia by inhibiting infection of type-1 alveloar epithelial cells. *PLoS Pathog,* 2017, vol. 13 (1), e1006140
- **CHEN, K.G. et al.** Human pluripotent stem cell culture: considerations for maintenance, expansion, and therapeutics. *Cell stem cell,* 2014, vol. 14, 13-26
- **CHOI, K.D. et al.** Generation of mature human myelomonocytic cells through expansion and differentiation of pluripotent stem cell-derived lin-CD34+CD43+CD45+ progenitors. *The Journal of clinical investigation,* 2009, vol. 119, 2818-2829
- **DIAS, J. et al.** Generation of red blood cells from human induced pluripotent stem cells. *Stem cells and development,* 2011, vol. 20, 1639-1647
- **DUTOW, P. et al.** Severity of allergic airway disease due to house dust mite allergen is not increased after clinical recovery of lung infection with Chlamydia pneumoniae in mice. *Infection and immunity,* 2013, vol. 81, 3366-3374
- **FALGENHAUER et al.** Colistin resistance gene mcr-1 in extended-spectrum beta-lactamase-producing and carbapenemase-producing Gram-negative bacteria in Germany. *Lancet Infect Dis,* 2016, vol. 16, 282-283
- **FENG, Q. et al.** Scalable generation of universal platelets from human induced pluripotent stem cells. *Stem cell reports,* 2014, vol. 3, 817-831
- **FREIRE-MORAN et al.** Critical shortage of new antibiotics in development against multidrug-resistant bacteria-Time to react is now. *Drug Resist Updat,* 2011, vol. 14, 118-124
- **GORDON, S.B. ; READ, R.C.** Macrophage defences against respiratory tract infections. *Br Med Bull,* 2002, vol. 61, 45-61
- **GOULD, I.M.** Treatment of bacteraemia: meticillin-resistant Staphylococcus aureus (MRSA) to vancomycin-resistant S. aureus (VRSA). *Int J Antimicrob Agents,* 2013, vol. 42, S17-21
- **HAPPLE, C. et al.** Pulmonary transplantation of macrophage progenitors as effective and long-lasting therapy for hereditary pulmonary alveolar proteinosis. *Science translational medicine,* 2014, vol. 6, 250ra113
- **HAUSER, A.R. et al.** Clinical significance of microbial infection and adaptation in cystic fibrosis. *Clin Microbiol Rev,* 2011, vol. 24, 29-70
- **HERIGSTAD, B. et al.** How to optimize the drop plate method for enumerating bacteria. *J Microbiol Methods,* 2001, vol. 44, 121-129
- **HIRSCH, E.B. ; TAM, V.H.** Impact of multidrug-resistant Pseudomonas aeruginosa infection on patient outcomes. *Expert Rev Pharmacoecon Outcomes Res,* 2010, vol. 10, 441-451
- **KEMPF, H. et al.** Large-scale production of human pluripotent stem cell derived cardiomyocytes. *Adv Drug Deliv Rev,* 2016, vol. 96, 18-30

- **KEMPF, H. et al.** Bulk cell density and Wnt/TGFbeta signalling regulate mesendodermal patterning of human pluripotent stem cells. *Nature communications,* 2016, vol. 7, 13602
- **KEMPF, H. et al.** Cardiac differentiation of human pluripotent stem cells in scalable suspension culture. *Nature protocols,* 2015, vol. 10, 1345-1361
- **KEMPF, H. et al.** Controlling expansion and cardiomyogenic differentiation of human pluripotent stem cells in scalable suspension culture. *Stem cell reports,* 2014, vol. 3, 1132-1146
- **KLOCKGETHER et al.** Genome diversity of Pseudomonas aeruginosa PAO1 laboratory strains. *J Bacteriol,* 2010, vol. 192, 1113-1121
- **KROPP, C. et al.** Impact of Feeding Strategies on the Scalable Expansion of Human Pluripotent Stem Cells in Single-Use Stirred Tank Bioreactors. *Stem cells translational medicine,* 2016, vol. 5, 1289-1301
- **KROPP, C. et al.** Progress and challenges in large-scale expansion of human pluripotent stem cells. *Process Biochemistry,* 2016
- **LACHMANN, N. et al.** Large-scale hematopoietic differentiation of human induced pluripotent stem cells provides granulocytes or macrophages for cell replacement therapies. *Stem cell reports,* 2015, vol. 4, 282-296
- **LACHMANN, N. et al.** Gene correction of human induced pluripotent stem cells repairs the cellular phenotype in pulmonary alveolar proteinosis. *American journal of respiratory and critical care medicine,* 2014, vol. 189, 167-182
- **LAVIN, Y. et al.** Tissue-resident macrophage enhancer landscapes are shaped by the local microenvironment. *Cell,* 2014, vol. 159, 1312-1326
- **LIU, Y.Y. et al.** Emergence of plasmid-mediated colistin resistance mechanism MCR-1 in animals and human beings in China: a microbiological and molecular biological study. *Lancet Infect Dis,* 2016, vol. 16, 161-168
- **MA, F. et al.** Generation of functional erythrocytes from human embryonic stem cell-derived definitive hematopoiesis. *Proceedings of the National Academy of Sciences of the United States of America,* 2008, vol. 105, 13087-13092
- **MIZGERD, J.P.** Respiratory infection and the impact of pulmonary immunity on lung health and disease. *Am J Respir Crit Care Med,* 2008, vol. 186, 824-829
- **MUNDER, A. et al.** Murine pulmonary infection with Listeria monocytogenes: differential susceptibility of BALB/c, C57BL/6 and DBA/2 mice. *Microbes Infect,* 2005, vol. 7, 600-611
- **NG, E.S. et al.** A protocol describing the use of a recombinant protein-based, animal product-free medium (APEL) for human embryonic stem cell differentiation as spin embryoid bodies. *Nature protocols,* 2008, vol. 3, 768-776
- **OLIVER, A. et al.** High frequency of hypermutable Pseudomonas aeruginosa in cystic fibrosis lung infection. *Science,* 2000, vol. 288, 1251-1254
- **OLIVER, A. et al.** The increasing threat of Pseudomonas aeruginosa high-risk clones. *Drug Resist Updat,* 2015, vol. 21-22, 41-59
- **OLMER, R. et al.** Suspension culture of human pluripotent stem cells in controlled, stirred bioreactors. *Tissue Eng Part C Methods,* 2012, vol. 18, 772-784
- **PASULA, R. et al.** Airway delivery of interferon-gamma overexpressing macrophages confers resistance to Mycobacterium avium infection in SCID mice. *Physiol Rep,* 2016, vol. 4
- **PINEROS, A.R. et al.** M2 macrophages or IL-33 treatment attenuate ongoing Mycobacterium tuberculosis infection. *Scientific Reports,* 2017, vol. 7
- **RAKHIMOVA, E. et al.** Pseudomonas aeruginosa population biology in chronic obstructive pulmonary disease. *The Journal of infectious diseases,* 2009, vol. 200, 1928-1935
- **RUEHL-FEHLERT, C. et al.** Revised guides for organ sampling and trimming in rats and mice--part 1. *Exp Toxicol Pathol,* 2003, vol. 55, 91-106
- **SCHROEDER, M.R. ; STEPHENS, D.S.** Macrolide Resistance in Streptococcus pneumoniae. *Front Cell Infect Microbiol,* 2016, vol. 6, 98
- **SHI, C. ; PAMER, E.G.** Monocyte recruitment during infection and inflammation. *Nature reviews Immunology,* 2011, vol. 11, 762-774
- **STURGEON, C.M. et al.** Wnt signaling controls the specification of definitive and primitive hematopoiesis from human pluripotent stem cells. *Nature biotechnology,* 2014, vol. 32, 554-561
- **SUZUKI, T. et al.** Pulmonary macrophage transplantation therapy. *Nature,* 2014, vol. 514, 450-454
- **TRAPNELL, B.C. et al.** Pulmonary alveolar proteinosis. *The New England journal of medicine,* 2003, vol. 349, 2527-2539
- **VAN DE LAAR, L. et al.** Yolk Sac Macrophages, Fetal Liver, and Adult Monocytes Can Colonize an Empty Niche and Develop into Functional Tissue-Resident Macrophages. *Immunity,* 2016, vol. 44, 755-768
- **VAN WILGENBURG, B. et al.** Efficient, long term production of monocyte-derived macrophages from human pluripotent stem cells under partly-defined and fully-defined conditions. *PloS one,* 2013, vol. 8, e71098
- **VENTOLA, C.L.** The antibiotic resistance crisis: part 1: causes and threats. *P T,* 2015, vol. 40, 277-283
- **WEISS, G. ; SCHAIBLE, U.E.** Macrophage defense mechanisms against intracellular bacteria. *Immunological reviews,* 2015, vol. 264, 182-203

- **WILLINGER, T. et al.** Human IL-3/GM-CSF knock-in mice support human alveolar macrophage development and human immune responses in the lung. *Proceedings of the National Academy of Sciences of the United States of America,* 2011, vol. 108, 2390-2395
- **WILLYARD, C.** The drug-resistant bacteria that pose the greatest health threats. *Nature,* 2017, vol. 543, 15
- **WOLBELING, F. et al.** Head-out spirometry accurately monitors the course of Pseudomonas aeruginosa lung infection in mice. *Respiration,* 2010, vol. 80, 340-346
- **ZHANG, H. et al.** Functional analysis and transcriptomic profiling of iPSC-derived macrophages and their application in modeling Mendelian disease. *Circulation research,* 2015, vol. 117, 17-28
- **ZWEIGERDT, R.** Large scale production of stem cells and their derivatives. *Adv Biochem Eng Biotechnol,* 2009, vol. 114, 201-235
- **ZWEIGERDT, R. et al.** Scalable expansion of human pluripotent stem cells in suspension culture. *Nature protocols,* 2011, vol. 6, 689-700